# EUROPEAN PATENT APPLICATION

(11) **EP 4 803 638 A1**
(43) Date of publication of application: **09.09.2026**
(21) Application number: 25382208.4
(22) Date of filing: 07.03.2025
(51) Int. Cl.: C12Q 1/6888

(54) **METHODS FOR DIAGNOSIS AND TREATING LIVER CANCER**

(71) Applicant: Asociación Centro de Investigación Cooperativa en Biociencias - CIC bioGUNE, 48160 Derio (ES)
(72) Inventor: MARTÍNEZ CHANTAR, María Luz, E-48160 Derio, Vizcaya (ES); ZAPATA PAVAS, Leidy Estefanía, E-48160 Derio, Vizcaya (ES)
(74) Representative: ABG Intellectual Property Law, S.L.

(57) **Abstract**

The present invention relates to a CAND1 inhibiting agent for use in the treatment of hepatoblastoma or hepatocellular carcinoma. Furthermore the invention relates to a method for the diagnosis of hepatoblastoma or hepatocellular carcinoma in a subject and to a method for determining the prognosis of a hepatoblastoma or hepatocellular carcinoma in a subject. A final aspect of the current invention is concerned with a method to determine the predisposition of a subject to developing hepatoblastoma or hepatocellular carcinoma.

## Description

### TECHNICAL FIELD OF THE INVENTION

The present invention relates to the field of liver cancer diagnosis and prognosis, as well as treating liver cancer.

### BACKGROUND OF THE INVENTION

Hepatoblastoma is the most common primary liver tumor in children aged 0 to 4 years. Currently the annual incidence of hepatoblastoma is 1.8 cases per million. The exact origin of hepatoblastoma is unknown and most cases are sporadic. It has been recently shown that DNA mutations, post-translational modifications as well as epigenetic changes contribute to the development of hepatoblastoma. Hepatoblastoma may remain asymptomatic for several months. Initial symptoms of hepatoblastoma includes painless abdominal swelling, while more advanced symptoms include developmental delays, jaundice and nausea. The diagnosis of hepatoblastoma relies on the detection of the levels of a series of biomarkers (e.g AFP, CK19, β-HCG, EpCAM) as well as confirmation by imagining techniques such as ultrasound, or Magnetic Resonance Imagining (MRI).

Currently, treatments for hepatoblastoma comprise chemotherapy, surgical resection and liver transplantation. Despite these treatments the rate of relapse is considerably high (around 12%). Furthermore, in such cases the tumors display resistance to chemotherapy which is correlated to poor prognosis.

Importantly, the complexity in the diagnosis of hepatoblastoma stems from its tumor heterogeneity and number of subtypes. Furthermore, at earlier stages, hepatoblastoma may remain asymptomatic. As such there is a growing need for developing new diagnosis tools enabling the detection of the disease as well as new treatments against hepatoblastoma. Additionally, there is also a need for the development of tools for prognosis of the disease, which will contribute to defining the most effective therapeutic interventions against hepatoblastoma.

### SUMMARY OF THE INVENTION

The inventors have found that in hepatoblastoma patients as well as mouse models of hepatoblastoma, the expression levels of the deNEDDylase, NEDP1, are drastically reduced (e.g. Figure 6). Consistently, restoring NEDP1 levels in mouse models of hepatoblastoma suppresses tumor cell growth and infiltration (e.g. Figure 25 and 26). The inventors report on a NEDD8-CAND1 axis inhibiting the tumor suppressing role exerted by NEDP1 (Figure 27). Finally, the inventors showed that hepatoblastoma or hepatocellular carcinoma patients displaying high levels of CAND1 have a reduced survival (Figure 34 and 35).

Thus, in a first aspect the inventions refers to a CAND1 inhibiting agent for use in the treatment of hepatoblastoma or hepatocellular carcinoma.

In another aspect the invention relates to an *in vitro* method for the diagnosis of hepatoblastoma or hepatocellular carcinoma in a subject, which comprises:
a) determining *CAND1* and/or *NEDP1* expression levels in a liver biopsy sample from a subject and
b) comparing these levels with a reference value, wherein increased expression levels of *CAND1* and/or decreased expression levels of NEDP1 compared with the reference value are indicative that the subject suffers from hepatoblastoma or hepatocellular carcinoma.

In yet another aspect, the invention relates to an *in vitro* method for the prognosis of a hepatoblastoma or a hepatocellular carcinoma in a subject which comprises:
a) determining *CAND1* and/or *NEDP1* expression levels in a liver biopsy sample from a subject and
b) comparing these levels with a reference value, wherein:
   - if the expression levels of CAND1 are increased compared to a reference value and/or,
   - if the expression levels of NEDP1 are decreased compared to a reference value
the patient has a poor prognosis.

In a final aspect the invention relates to an *in vitro* method for determining the predisposition of a subject to developing a hepatoblastoma or a hepatocellular carcinoma, the method comprising:
a) determining the deNEDDylation activity in a liver biopsy sample and
b) comparing these levels with a reference value,
wherein the decreased levels of deNEDDylation activity of the sample compared to a reference value are indicative of increased chances of developing a hepatoblastoma or a hepatocellular carcinoma.

### BRIEF DESCRIPTION OF THE DRAWINGS

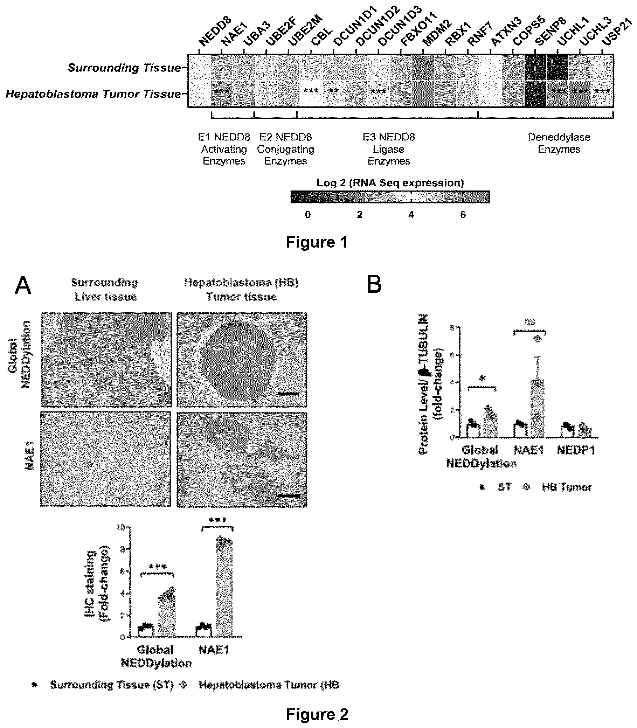

**Figure 1****.** Heat map from gene expression levels represented as a Log2 of the value of the 19 enzymes involved in NEDDylation pathway analyzed in a cohort of 32 hepatoblastoma patients in healthy surrounding tissue and tumor. One-way ANOVA test, was used to compare groups. **p<0.01 and ***p<0.001 are shown.
**Figure 2****.** A.Hepatic NEDDylation levels and NAE1 assessed by immunohistochemistry in Hepatoblastoma tumors in cohort of patients (n=4). Scale bar corresponds to 100 µm. B. Western blot analysis of global NEDDylated pattern, NEDD8 activating enzyme 1 (NAE1), NEDD8-Specific Protease 1 (NEDP1) and β-TUBULIN in a cohort of patients (n=3),

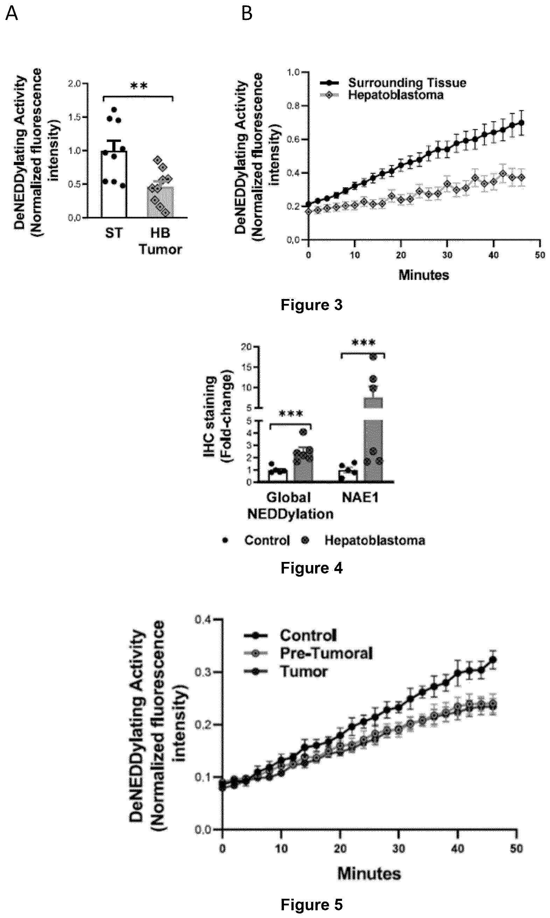

**Figure 3****. A.** Fold change in deNEDDylating activity levels in a patient cohort (n=9), all of them comparing tumor tissues to surrounding tissues. Data is shown as average ± SEM. Student's t-test respectively, was used to compare groups. **p<0.01.
   B. Kinetic profile of deNEDDylating activity levels expressed as normalized fluorescence activity in a patient cohort (n=9) comparing tumor tissues to surrounding tissues.
**Figure 4****.** Quantification of hepatic NEDDylation levels and NAE1 assessed by immunohistochemistry in mouse models of HB comparing healthy liver (n=6) and tumoral state after 11 weeks of tumor induction (n=6). Data is shown as average ± SEM. Student's t-test was used to compare groups. ***p<0.001 are shown.
**Figure 5****.** Kinetic profile of deNEDDylating activity levels expressed as normalized fluorescence activity in a mouse mice model of Hepatoblastoma comparing healthy liver (n=4), pre-tumoral state after 6 weeks (n=4) and tumoral state after 11 weeks of tumor induction (n=4).

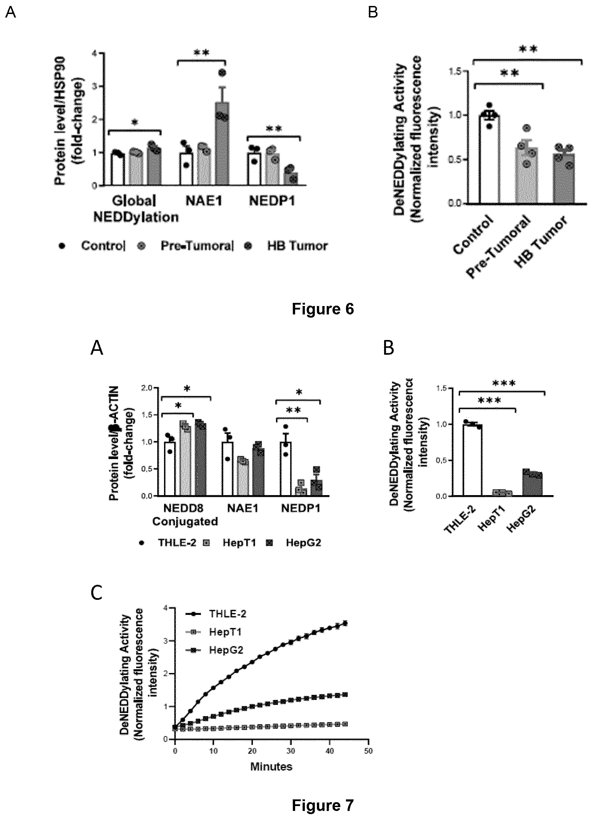

**Figure 6****. A.** Quantification of Western blot analysis of global NEDDylated pattern, NEDD8 activating enzyme 1 (NAE1), NEDD8-Specific Protease 1 (NEDP1) and Heat Shock Protein 90A (HSP90) (used as loading control). Data is shown as average ± SEM. One-way ANOVA test, was used to compare groups. *p<0.05, **p<0.01 are shown.
   B. Fold change of deNEDDylating activity levels in an *in vivo* mouse model of Hepatoblastoma comparing healthy liver (n=4), pre-tumoral state after 6 weeks (n=4) and tumoral state after 11 weeks of tumor induction (n=4). Data is shown as average ± SEM One-way ANOVA test, was used to compare groups. **p<0.01 is shown.
**Figure 7****. A.** Quantification of Western blot analysis of global NEDDylated pattern, NEDD8 activating enzyme 1 (NAE1), NEDD8-Specific Protease 1 (NEDP1) and β-ACTIN (used as loading control). Data is shown as average ± SEM One-way ANOVA test, was used to compare groups. *p<0.05, **p<0.01 are shown.
   B. Fold change of deNEDDylating activity levels, in an *in vitro* pre-clinical model of cell lines including THLE-2, consider healthy hepatocytes, HepT1 and HepG2 hepatoblastoma cancer cells. Data is shown as average ± SEM One-way ANOVA test, was used to compare groups. ***p<0.001 are shown.
   C. Kinetic profile of deNEDDylating activity levels expressed as normalized fluorescence activity.

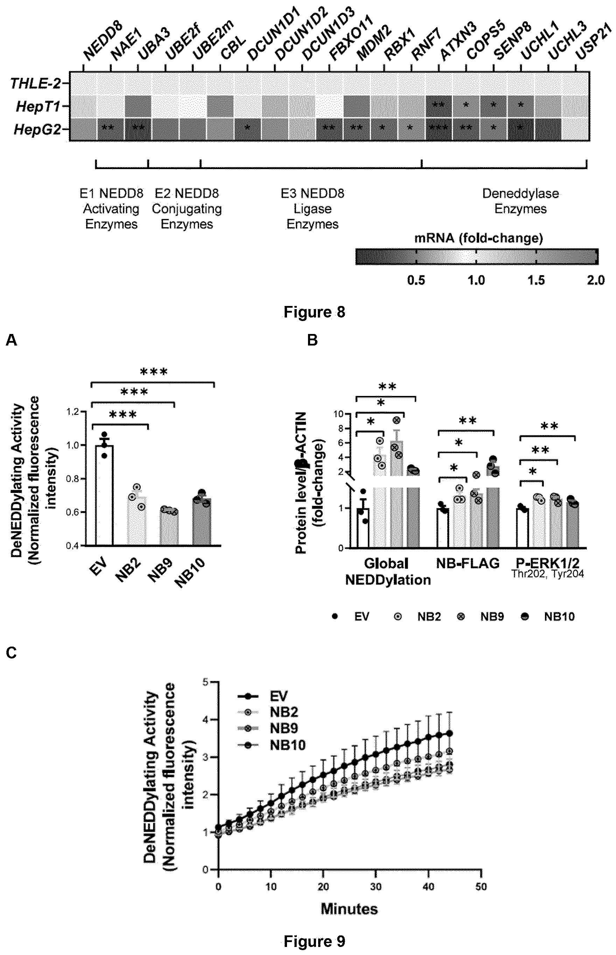

**Figure 8****.** mRNA expression levels of NEDD8 pathway (NEDD8, UBA3, NAE-1, UBE2M, UBE2F, CBL, DCUND1D1, DCUND1D2, DCUN1D3, FBXO11, MDM2, RBX1, RNF7, ATXN3, SENP8, COPS5, UCHL3, UCHL1 AND USP21) at *in vitro* pre-clinical model comprised THLE-2 (healthy hepatocytes) along with HepT1 and HepG2 hepatoblastoma cancer cell lines. One-way ANOVA test was used to compare groups. *p<0.05, **p<0.01 and ***p<0.001 are shown.
**Figure 9****.** A. Fold change of deNEDDylating activity levels in THLE-2, non tumoral cells, transfected with empty vector (EV), NB2, NB9 and NB10 for 72 hours. Data is shown as average ± SEM. One-way ANOVA test was used to compare groups. *p<0.05, **p<0.01 and ***p<0.001 are shown. B. Quantification of Western blot analysis of NEDDylated cullins, Sigma-Flag associated with NB, phospho Extracellular Signal-Regulated Kinase 1 and 2 (Phospho-ERK1/ERK2 Thr202, Tyr204) and β-ACTIN (as loading control). Data is shown as average ± SEM. One-way ANOVA test was used to compare groups. *p<0.05, **p<0.01 are shown. C.Kinetic profile of deNEDDylating activity levels expressed as normalized fluorescence activity THLE-2, non-tumoral cells, transfected with empty vector (EV), NB2, NB9 and NB10 for 72 hours.

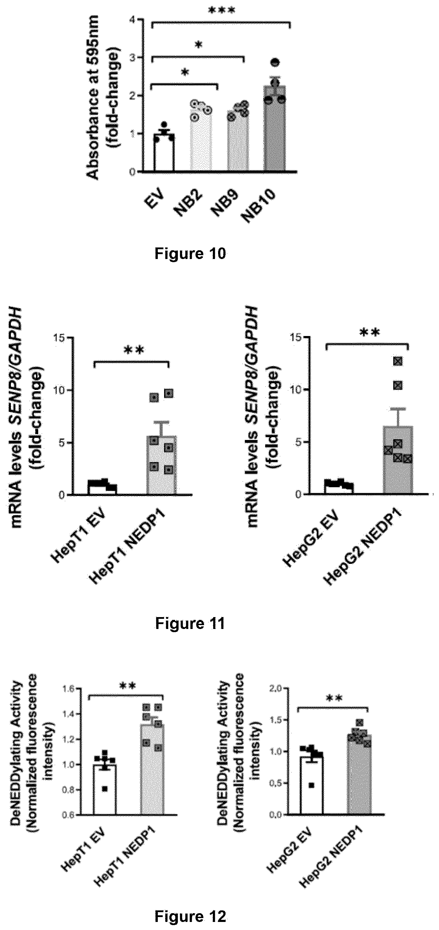

**Figure 10****.** Crystal Violet viability assay in THLE-2, non-tumoral cells, transfected with empty vector (EV), NB2, NB9 and NB10 for 72 hours. Data is shown as average ± SEM. One-way ANOVA test, was used to compare groups. *p<0.05 and ***p<0.001 are shown.
**Figure 11****.** mRNA analysis of SENP8, gene encoding NEDP1. Data is shown as average ± SEM. Student's t-test respectively, was used to compare groups. **p<0.01 is shown.
**Figure 12****.** Fold change of NEDP1 activity levels evaluated for both HepT1 and HepG2 cell lines transfected with Empty vector (EV) and NEDP1 for 48 hours. Student's t-test respectively, was used to compare groups. **p<0.01 is shown.

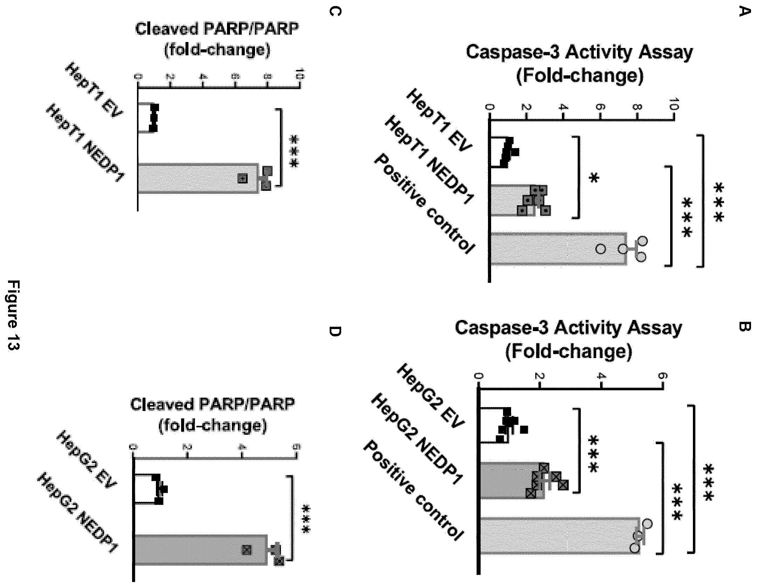

**Figure 13****. A.** Caspase-3 activity in HepT1 (A) and HepG2 (B) cell lines after 48 hours of EV or NEDP1 transfection. Data is shown as average ± SEM. One-way ANOVA test was used to compare groups. *p<0.05, ***p<0.001 are shown. Quantification of Western blot analysis of Poly (ADP-Ribose) Polymerase 1 (PARP), PARP cleavage and β-actin (as loading control) in HepT1 (C) and HepG2 (D) cell lines after 48 hours of EV or NEDP1 transfection. Data is shown as average ± SEM. Student's t-test was used to compare groups. ***p<0.001 is shown.

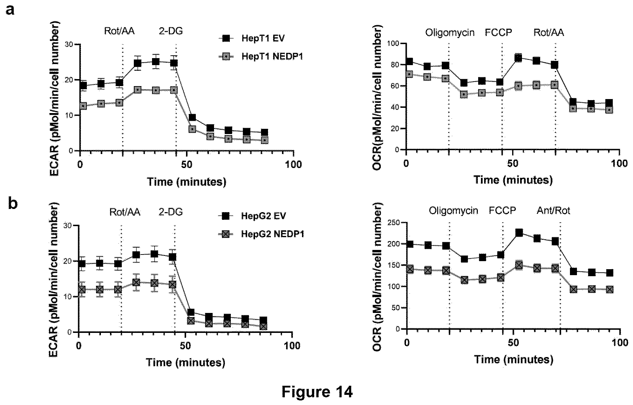

**Figure 14****. A.** Kinetic profiles of Oxygen Consumption Rate (OCR) and Extracellular Acidification Rate (ECAR) in the HepT1 cell line transfected with Empty vector (EV) and NEDP1 for 48 hours. B. Kinetic profiles of Oxygen Consumption Rate (OCR) and Extracellular Acidification Rate (ECAR) in the HepG2 cell line transfected with Empty vector (EV) and NEDP1 for 48 hours.

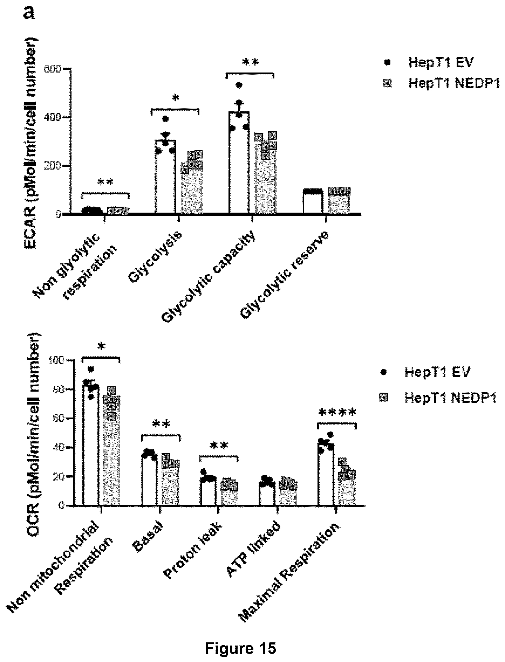

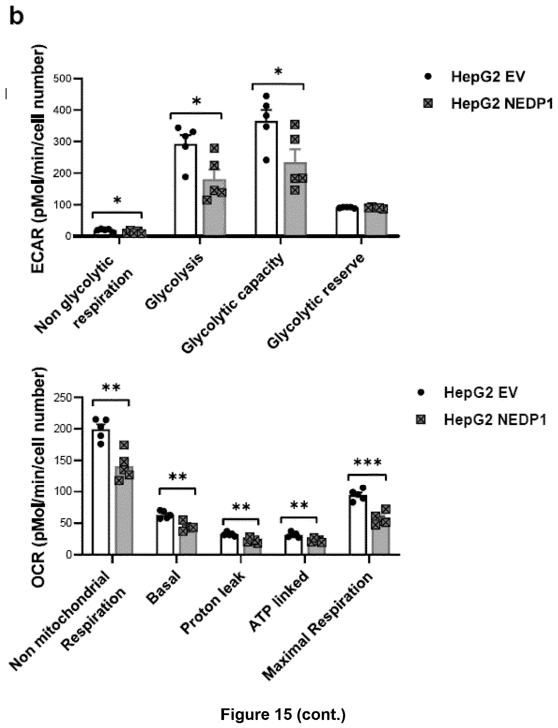

**Figure 15****. A.** Extra Cellular Acidification Rate (ECAR), and Oxygen Consumption Rate (OCR) measurements in HepT1 cell line transfected with Empty vector (EV) and NEDP1 for 48 hours. Data is shown as average ± SEM. Student's t-test respectively, was used to compare groups. *p<0.05, **p<0.01 and ****p<0.0001 are shown. B. Extra Cellular Acidification Rate (ECAR), and Oxygen Consumption Rate (OCR) measurements in HepG2 transfected with Empty vector (EV) and NEDP1 for 48 hours. Data is shown as average ± SEM. Student's t-test, was used to compare groups. *p<0.05, **p<0.01 and ***p<0.001 are shown.

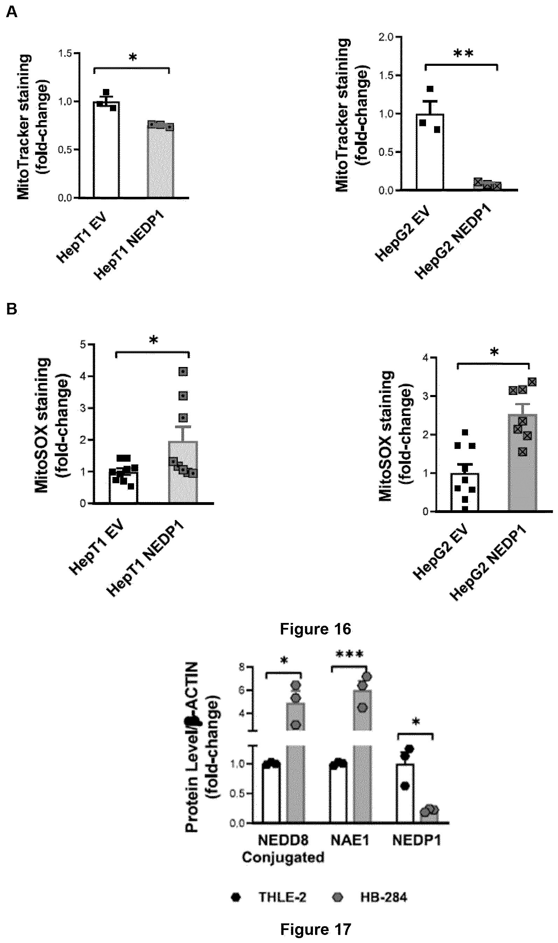

**Figure 16****. A.** Quantification of mitochondrial total number (MitoTracker) evaluated for both HepT1 and HepG2 cell lines transfected with Empty vector (EV) and NEDP1 for 48 hours. Scale bar corresponds to 100 µm. Data is shown as average ± SEM. Student's t-test was used to compare groups. *p<0.05, **p<0.01 are shown. B. Quantification of mitochondrial fluorescent marker of reactive oxygen species (ROS, MitoSOX) evaluated for both HepT1 and HepG2 cell lines transfected with Empty vector (EV) and NEDP1 for 48 hours. Scale bar corresponds to 100 µm. Data is shown as average ± SEM. Student's t-test was used to compare groups. *p<0.05, is shown.
**Figure 17****.** Quantification of Western blot analysis of global NEDDylated pattern, NEDD8 activating enzyme 1 (NAE1), NEDD8-Specific Protease 1 (NEDP1) and β-actin (as loading control) in PDX (HB-284) cells and THLE-2 cells (healthy hepatocytes). Data is shown as average ± SEM. Student's t-test was used to compare groups. *p<0.05, ***p<0.001 are shown.

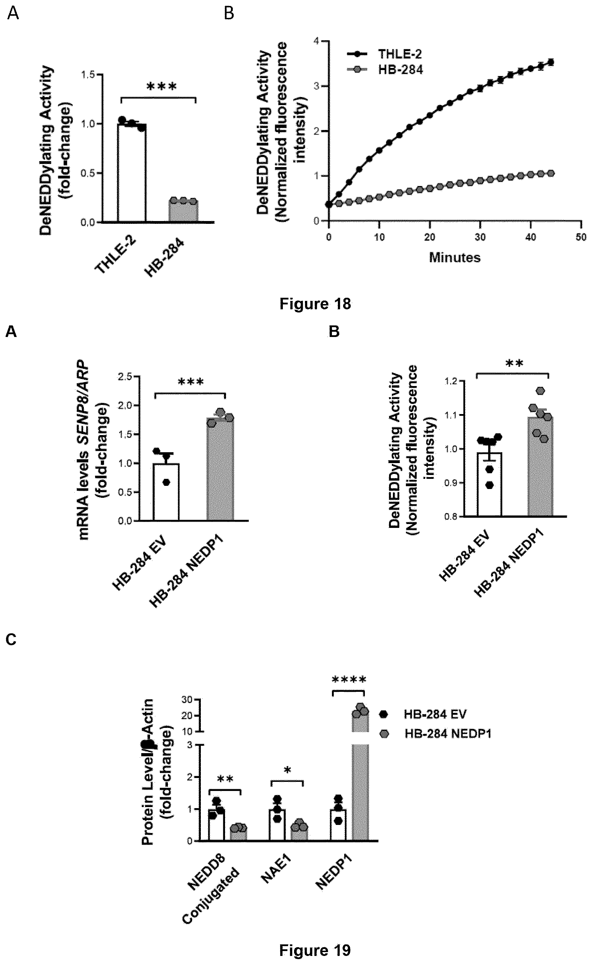

**Figure 18****.** A. Fold change of deNEDDylating activity levels in PDX (HB-284) cells and THLE-2 cells (healthy hepatocytes). Data is shown as average ± SEM. Student's t-test was used to compare groups. ***p<0.001 is shown. B. Kinetic profile of deNEDDylating activity levels expressed as normalized fluorescence activity in PDX (HB-284) cells and THLE-2 cells (healthy hepatocytes).
**Figure 19****.** A. mRNA analysis of SENP8, gene encoding NEDP1 in PDX (HB-284) cell line transfected with Empty vector (EV) and NEDP1 for 48 hours. Data is shown as average ± SEM. Student's t-test was used to compare groups. ***p<0.001 is shown. B. Fold change of deNEDDylating activity levels in PDX (HB-284) cell line transfected with Empty vector (EV) and NEDP1 for 48 hours. Data is shown as average ± SEM. Student's t-test was used to compare groups. **p<0.01 is shown. C. Quantification of Western blot analysis of global NEDDylated pattern, NEDD8 activating enzyme 1 (NAE1), NEDD8-Specific Protease 1 (NEDP1) and β-actin (as loading control) in PDX (HB-284) cell line transfected with Empty vector (EV) and NEDP1 for 48 hours. Data is shown as average ± SEM. Student's t-test was used to compare groups. *p<0.05, **p<0.01 and ****p<0.0001 are shown.

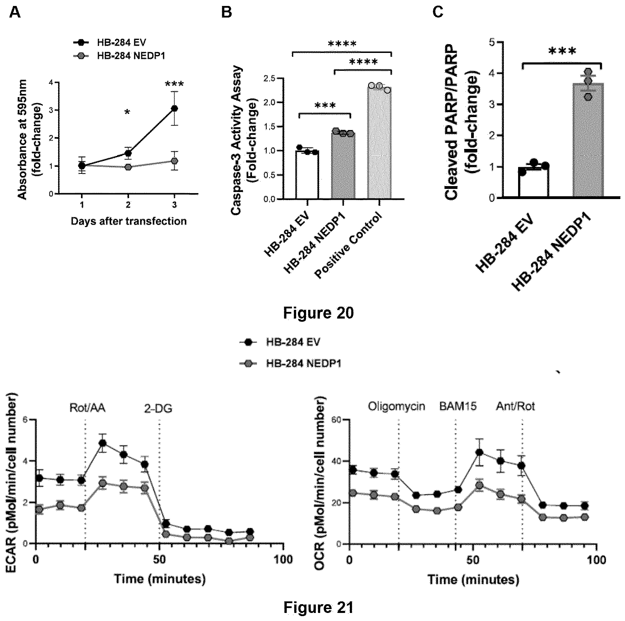

**Figure 20****. A.** Crystal Violet viability assay evaluated for PDX (HB-284) cell line transfected with Empty vector (EV) and NEDP1 for 48 hours. Data is shown as average ± SEM. Student's t-test was used to compare groups. *p<0.05 and ***p<0.001 are shown. **B.** Caspase-3 activity, evaluated for PDX (HB-284) cell line transfected with Empty vector (EV) and NEDP1 for 48 hours. Data is shown as average ± SEM. Student's t-test was used to compare groups. ***p<0.001 and ****p<0.0001 are shown. C. Quantification of Western blot analysis of Poly (ADP-Ribose) Polymerase 1 (PARP), PARP cleavage and β-ACTIN (as loading control) evaluated for PDX (HB-284) cell line transfected with Empty vector (EV) and NEDP1 for 48 hours. Data is shown as average ± SEM. Student's t-test was used to compare groups. ***p<0.001 is shown.
**Figure 21****.** Kinetic profiles of Extracellular Acidification Rate (ECAR) and Oxygen Consumption Rate (OCR) evaluated for PDX (HB-284) cell line transfected with Empty vector (EV) and NEDP1 for 48 hours.

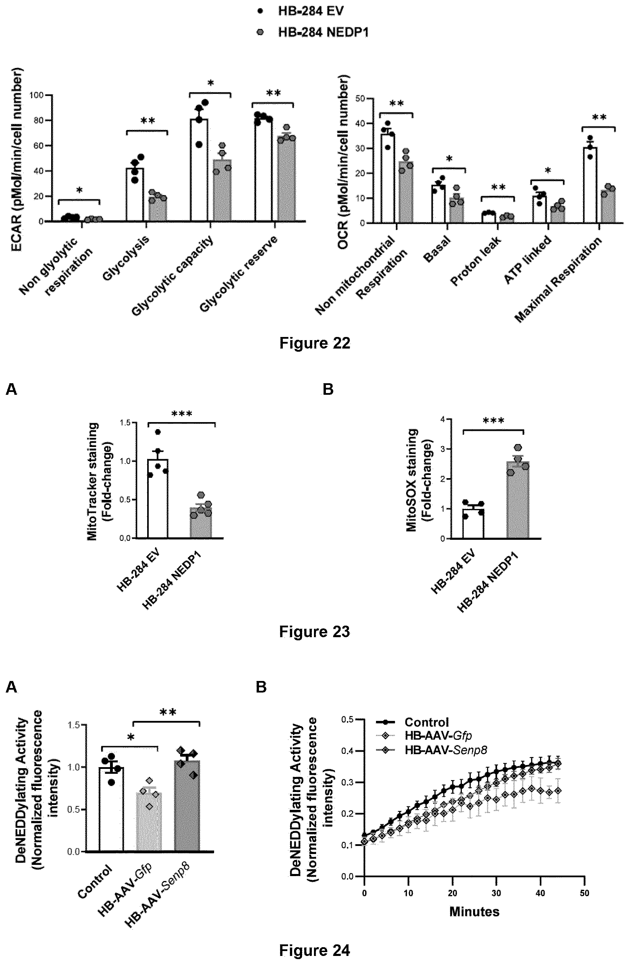

**Figure 22****.** Extra Cellular Acidification Rate (ECAR), and Oxygen Consumption Rate (OCR) measurements in PDX (HB-284) cell line transfected with Empty vector (EV) and NEDP1 for 48 hours. Data is shown as average ± SEM. Student's t-test was used to compare groups. *p<0.05, **p<0.01 are shown.
**Figure 23****. A.** Quantification of Mitochondrial fluorescent marker (MitoTracker) evaluated for PDX (HB-284) cell line transfected with Empty vector (EV) and NEDP1 for 48 hours. Scale bar corresponds to 100 µm. Data is shown as average ± SEM. Student's t-test was used to compare groups. ***p<0.001 is shown. B. Quantification of Mitochondrial fluorescent marker of reactive oxygen species (ROS, MitoSOX) evaluated for PDX (HB-284) cell line transfected with Empty vector (EV) and NEDP1 for 48 hours. Data is shown as average ± SEM. Student's t-test was used to compare groups. ***p<0.001 is shown. **Figure 24****.** A. Fold change of deNEDDylating activity levels on healthy mice (n=4) compared to Hepatoblastoma model in ^{Bio}NEDD8 mice treated with adenoviruses HB-AAV-Gfp (n=4) or HB-AAV-Senp8 (n=4). **p<0.01 and ***p<0.001 are shown. B. Kinetic profile of deNEDDylating activity levels expressed as normalized fluorescence activity in a control group (n=4) (C57BL/6J mice), and in a hepatoblastoma mouse model treated with adenovirus HB-AAV-Gfp (n=4) or HB-AAV-Senp8 (n=4).

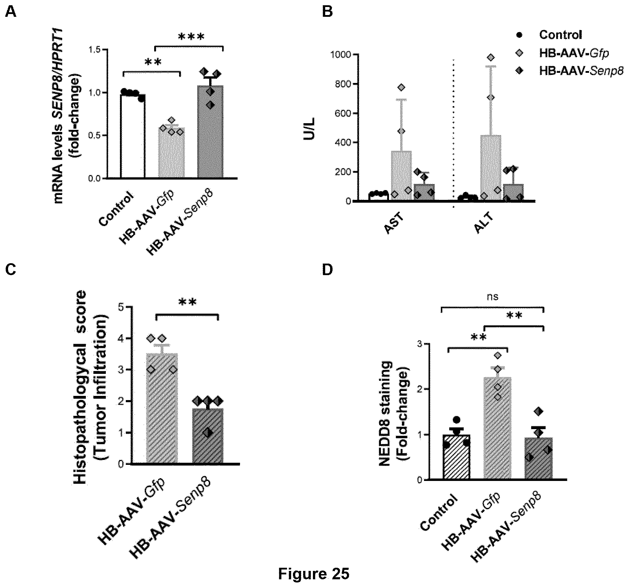

**Figure 25****. A.** mRNA analysis of SENP8, gene name of NEDP1 protein in a control group (n=4) (C57BL/6J mice), and in a hepatoblastoma mouse model treated with adenovirus HB-AAV-Gfp (n=4) or HB-AAV-Senp8 (n=4). Data is shown as average ± SEM. One-way ANOVA test was used to compare groups. **p<0.01 and ***p<0.001 are shown. B. Basal serum levels of AST and ALT in C57BL/6J mice model hepatoblastoma model comparing HB mice treated with adenovirus HB-AAV-Gfp (n=4) or HB-AAV-Senp8 (n=4) with control group (n=4). Data is shown as average ± SEM. C. Quantitative histopathological analysis and H&E liver immunohistochemical staining on hepatoblastoma model treated with adenovirus HB-AAV-Gfp (n=4) or HB-AAV-Senp8 (n=4) in C57BL/6J mice model. **p<0.01 is shown. D. Hepatic NEDDylation levels assessed by immunohistochemistry on healthy liver (n=4) and hepatoblastoma model treated with adenovirus HB-AAV-Gfp (n=4) or HB-AAV-Senp8 (n=4) in C57BL/6J mice model. Data is shown as average ± SEM. One-way ANOVA test was used to compare groups. *p<0.05, **p<0.01 and ****p<0.0001 are shown.

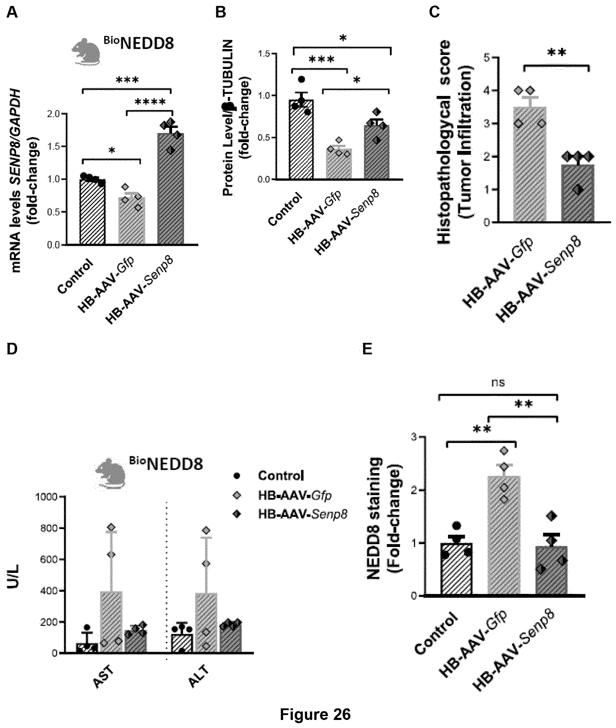

**Figure 26****. A.** mRNA analysis of SENP8, gene name of NEDP1 on healthy mice (n=4) compared to Hepatoblastoma model in ^{Bio}NEDD8 mice treated with adenoviruses HB-AAV-Gfp (n=4) or HB-AAV-Senp8 (n=4). Data is shown as average ± SEM. One-way ANOVA test was used to compare groups. *p<0.05, ***p<0.001 and ****p<0.0001 are shown. B. Quantification of Western blot analysis of NEDD8-Specific Protease 1 (NEDP1) and β-Tubulin (as loading control), on healthy mice (n=4) compared to HB model in ^{Bio}NEDD8 mice treated with adenoviruses HB-AAV-Gfp (n=4) or HB-AAV-Senp8 (n=4). Data is shown as average ± SEM. One-way ANOVA test was used to compare groups. *p<0.05 and ***p<0.001 are shown. C. Quantitative histopathological analysis and H&E liver immunohistochemical staining, on healthy mice (n=4) compared to Hepatoblastoma model in ^{Bio}NEDD8 mice treated with adenoviruses HB-AAV-Gfp (n=4) or HB-AAV-Senp8 (n=4). Data is shown as average ± SEM. One-way ANOVA test was used to compare groups. **p<0.01 is shown. D. Basal serum levels of AST and ALT, on healthy mice (n=4) compared to HB model in ^{Bio}NEDD8 mice treated with adenoviruses HB-AAV-*Gfp* (n=4) or HB-AAV-Senp8 (n=4). Data is shown as average ± SEM. E. Hepatic NEDDylation levels assessed by immunohistochemistry on healthy mice (n=4) compared to Hepatoblastoma model in ^{Bio}NEDD8 mice treated with adenoviruses HB-AAV-Gfp (n=4) or HB-AAV-Senp8 (n=4). Data is shown as average ± SEM. One-way ANOVA test was used to compare groups. **p<0.01 and ns (not significant) are shown.

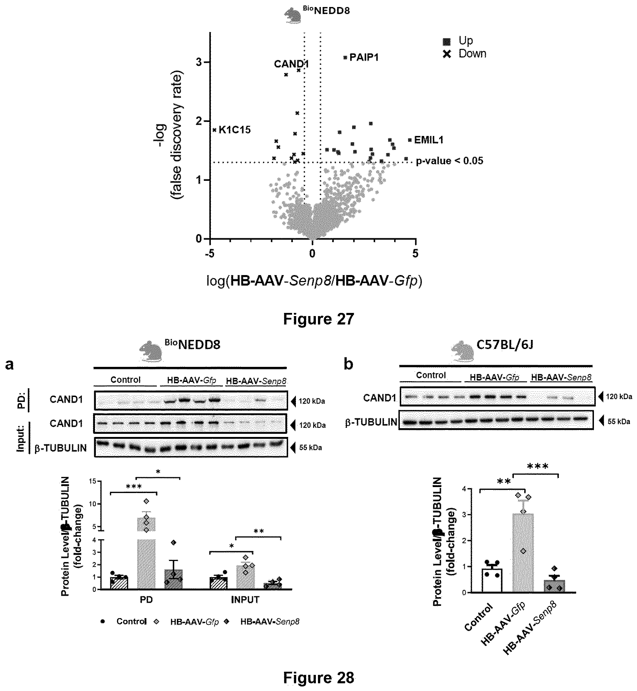

**Figure 27****.** Volcano plot of proteins representing the Log 2 of the fold-change ratio and - Log of the p-value, squares and crosses represent all of the considered proteins for the analysis, after pull-down enrichment of biotinylated proteins in ^{Bio}NEDD8 mice treated with HB-AAV-Gfp (n=3) compared to mice treated with HB-AAV-Senp8 (n=3).
**Figure 28****. A.** Quantification of Protein expression levels of Cullin Associated and NEDDylation Dissociated 1 (CAND1) after pull-down enrichment of biotinylated proteins and in the input extracts from hepatoblastoma ^{Bio}NEDD8 mice treated with HB-AAV-Gfp (n=4) and HB-AAV-Senp8 (n=4) compared with a control group (n=4). β-Tubulin was used as a loading control from the input protein extract. Data is shown as average ± SEM. One-way ANOVA test was used to compare groups. *p<0.05, **p<0.01, ***p<0.001 are shown. **B.** Quantification of Western blot analysis of protein expression levels of CAND1 in total liver homogenate of from hepatoblastoma C57BL/6J mice treated with HB-AAV-Gfp (n=4) and HB-AAV-Senp8 (n=4) compared with a control group (n=4). β-Tubulin was used as a loading control. Data is shown as average ± SEM. One-way ANOVA test and Student's t-test respectively, was used to compare groups. **p<0.01, ***p<0.001 are shown.

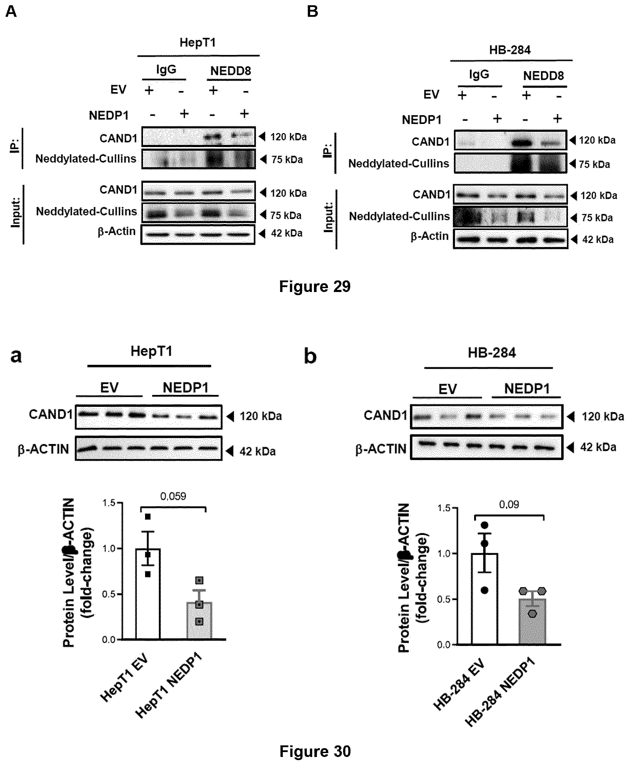

**Figure 29****. A.** Western blot analysis of CAND1, cullin NEDDylated pattern and β-actin obtained from the immunoprecipitation of NEDD8 and input samples in HepT1 cell lines transfected with Empty vector (EV) and NEDP1 for 48 hours. B. Western blot analysis of CAND1, cullin NEDDylated pattern and β-actin obtained from the immunoprecipitation of NEDD8 and input samples in PDX (HB-284) cell lines transfected with Empty vector (EV) and NEDP1 for 48 hours.
**Figure 30****. A.** Western blot analysis and quantification of Cullin Associated and NEDDylation Dissociated 1 (CAND1) and β-actin in HepT1 cell lines transfected with Empty vector (EV) and NEDP1 for 48 hours. Data is shown as average ± SEM. Student's t-test was used to compare groups. **B.** Western blot analysis and quantification of Cullin Associated and NEDDylation Dissociated 1 (CAND1) and β-actin in PDX (HB-284) cell lines transfected with Empty vector (EV) and NEDP1 for 48 hours. Data is shown as average ± SEM. Student's t-test was used to compare groups.

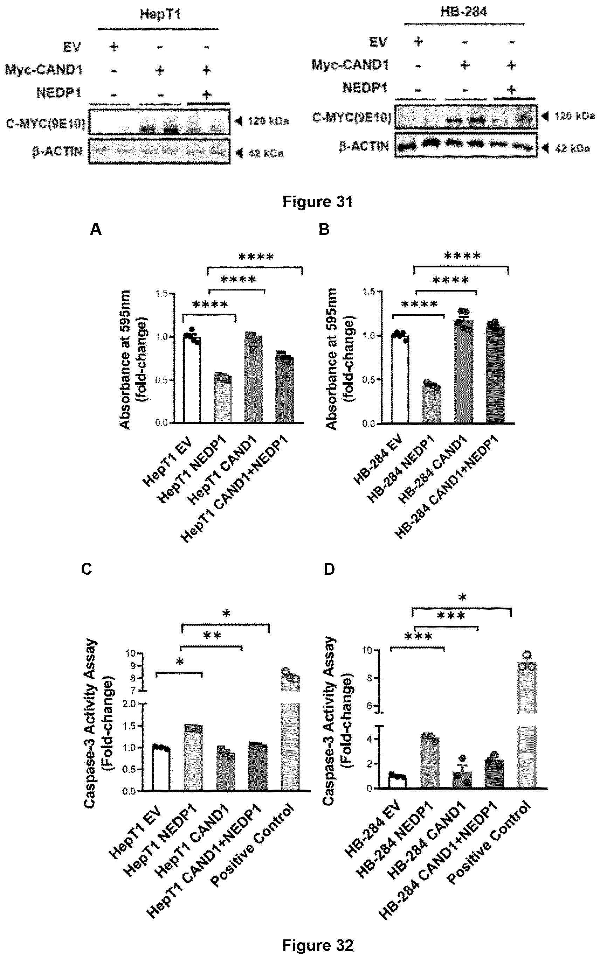

**Figure 31****.** Western blot analysis of Cullin Associated and NEDDylation Dissociated 1 (CAND1) and β-ACTIN in HepT1 **(A)** and PDX (HB-284) **(B)** cell lines transfected with Empty vector (EV), CAND1 and co-transfected with NEDP1 and CAND1 for 72 hours.
**Figure 32****.** Crystal Violet viability assay in HepT1 (A) and PDX (HB-284) (B) cell lines transfected with Empty vector (EV), NEDP1, CAND1 and co-transfected with NEDP1 and CAND1 for 72 hours. Data is shown as average ± SEM. One-way ANOVA test and Student's t-test respectively, was used to compare groups. ****p<0.0001 is shown. Caspase-3 activity in HepT1 (C) and PDX (HB-284) (D) cell lines transfected with Empty vector (EV), NEDP1, CAND1 and co-transfected with NEDP1 and CAND1 for 72 hours. Data is shown as average ± SEM. One-way ANOVA test was used to compare groups. *p<0.05, **p<0.01 and ***p<0.001 are shown.

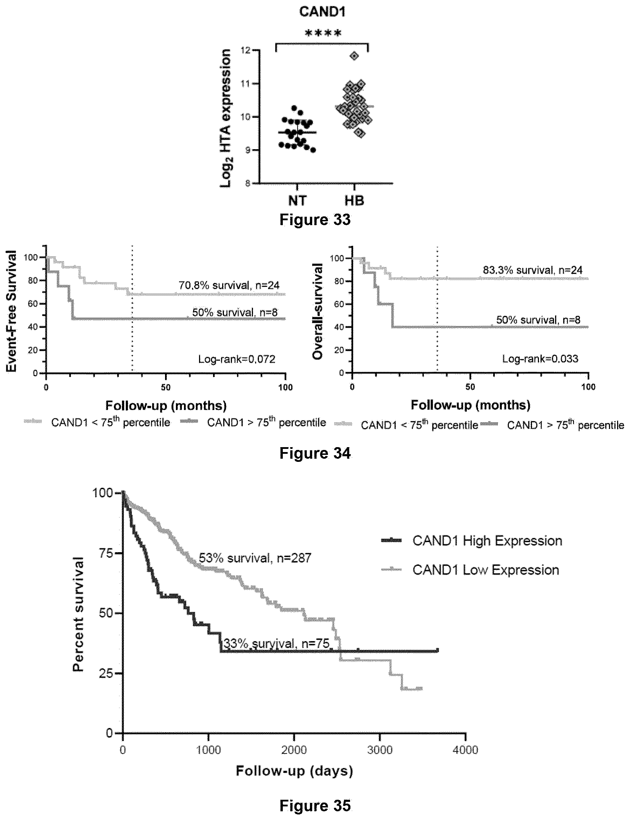

**Figure 33****.** HTA Gene expression of CAND1 in adjacent non-tumor livers (NT, n=20) and Hepatoblastoma (HB, n=32). Data is shown as average ± SEM. One-way ANOVA test was used to compare groups. ****p<0.0001 is shown.
**Figure 34****.** Event-free survival (left) and Overall survival (right) Kaplan- Meier survival curve of patients categorized according to CAND1 gene expression by HTA array.
**Figure 35****.** Kaplan-Meir survival curve for overall survival of patients based on CAND1 mRNA expression in Hepatocellular Carcinoma patients. Data retrieved from the Human Protein Atlas. Patients were stratified into high (>15.38) and low (<15.38) expression groups based on TPM (Transcripts Per Million). Median survival was 765 days for the high-expression group and 2116 days for the low-expression group. Statistical analysis was performed using the Log-rank (Mantel-Cox) test, showing a significant difference between the curves (Chi-square = 14.90, df = 1, p =0.0001).

### DETAILED DESCRIPTION OF THE INVENTION

As previously mentioned, the inventors have found that in patients suffering from hepatoblastoma and mouse models of hepatoblastoma, NEDP1 levels are drastically reduced. Consistently, restoring NEDP1 levels in mouse models of hepatoblastoma suppresses tumor cell growth and infiltration. Furthermore, it is shown that hepatoblastoma or hepatocellular carcinoma patients displaying high levels of CAND1 have a reduced survival.

### Medical use of the invention

In a first aspect the present invention relates to an agent capable of inhibiting the expression of Cullin Associated and Neddylation Dissociated 1 (CAND1) and/or inhibiting the activity of the protein encoded by said gene in the treatment and/or prevention of hepatoblastoma (HB) or hepatocellular carcinoma (HCC).

Alternatively, the first aspect of the invention relates to the use of an agent capable of inhibiting the expression of *CAND1* and/or inhibiting the activity of the protein encoded by said gene for the manufacture of a medicament for the treatment and/or prevention of hepatoblastoma (HB) or hepatocellular carcinoma (HCC).

Alternatively, the fist aspect of the invention relates to a method of treating and/or preventing hepatoblastoma (HB) or hepatocellular carcinoma (HCC), comprising administering an agent capable of inhibiting the expression of *CAND1* and/or inhibiting the activity of the protein encoded by said gene to a patient in need thereof.

The term "Cullin Associated and Neddylation Dissociated 1" or *"CAND1",* as used herein, refers to a protein coding-gene. *CAND1* is essential in the regulation of cullin-NEDDylation and the formation of an active Cullin Ring Ligase complex. Specifically, CAND1 plays a relevant role in the regulation of the Skip-cullin 1-F box (SCF) function. The binding between CAND1 and cullin1 leads to different conformations that facilitate the assembly and disassembly of the SCF complex. The human *CAND1* corresponds to the gene identified in the NCBI database by the gene ID 55832 (release date 9 February 2025).

There are 4 different CAND1 isoforms encoded by 4 different mRNA sequences. These sequences are defined in NCBI with the entry NM_001329674.2, NM_001329675.2, NM_001329676.2 and NM_018448.5.

The protein encoded by *CAND1* is called "Cullin-associated NEDD68-dissociated protein 1" or "CAND1". In a particular embodiment CAND1 is human CAND1 as defined in the UniProt database with the accession number Q86VP6, entry version 198, sequence version 2, release date 5 February 2025.

The term "agent capable of inhibiting the expression" is understood as any substance or compound which is capable of specifically silencing, reducing, preventing and/or blocking the expression of the gene, either by preventing the transcription of the gene *CAND1,* therefore avoiding the formation of any of the transcriptional products of the gene, or by promoting the degradation of any of the transcriptional products of the gene *CAND1* or by specifically reducing, preventing and/or blocking the production of the encoded protein. The term "agent capable of inhibiting the activity" is understood as any substance or compound which is capable of specifically inhibiting the activity of the CAND1 protein.

The term "treatment" is understood as comprising any type of therapy which aims at terminating, ameliorating and/or reducing the susceptibility to a clinical condition as described herein. Thus, "treatment" "treating" and the like, as used herein, refer to obtaining a desired pharmacologic and/or physiologic effect, covering any treatment of a pathological condition or disorder in a mammal, including a human. The effect may be therapeutic in terms of a partial or complete cure for a disorder and/or adverse effect attributable to the disorder. It covers any treatment of a disease in a mammal, particularly in a human, and includes: (a) increasing survival time; (b) decreasing the risk of death due to the disease; (c) inhibiting the disease, i.e., arresting its development (e.g., reducing the rate of disease progression); and (d) relieving the disease, i.e., causing regression of the disease.

The term "prevention", "preventing" or the like as used here refer to any type of therapy which aims at obtaining a desired pharmacological and/or physiological effect, covering any treatment of a pathological condition or disorder in a mammal, including human. The effect may be prophylactic in terms of completely or partially preventing a disorder or symptom thereof. It covers any treatment of a disease in a mammal, particularly in a human, and includes preventing the disease from occurring in a subject which may be predisposed to the disease but has not yet been diagnosed as having it.

The term "subject"" "patient" or "individual" are used herein interchangeably to refer to any member of the animal kingdom and can be a vertebrate, such as a mammal, a fish, a bird, a reptile, or an amphibian, including a human, non-human primate, horse, pig, rabbit, dog, sheep, goat, cow, cat, guinea pig or rodent. Preferably, the subject is mammal, more preferably a human.

The term "hepatoblastoma" or "HB" refers to a rare pediatric liver tumor, predominantly affecting the population under three years of age. Hepatoblastoma is the most common primary liver tumor in childhood, ages 0 to 4. The current incidence of hepatoblastoma is at 4 cases per million children, aged 0 to 4 years, annually. Hepatoblastoma accounts for 67 to 80% of pediatric liver cancers worldwide. The standard treatment for hepatoblastoma comprises surgical resection, cisplatin-based chemotherapy and/or doxorubicin-based chemotherapy. A significant percentage of hepatoblastoma patients suffer from tumor relapse, or display enhanced resistance to chemotherapy leading to a poor prognosis.

The term "hepatocellular carcinoma or "HCC" refers to the most common type of liver cancer affecting adults, accounting for approximately 90% of liver cancer cases. The main risk factors for developing hepatocellular carcinoma are infection by the hepatitis B virus or by the hepatitis C virus. Additionally, development of hepatocellular carcinoma has been linked to non-alcoholic steatohepatitis associated with metabolic syndrome or diabetes mellitus. The diagnosis relies on the combination of measurement of biomarkers such as alpha-fetoprotein with imaging techniques such as ultrasound or magnetic resonance imagining (MRI).

In a particular embodiment the agent capable of inhibiting the expression of Cullin Associated and Neddylation Dissociated 1 (CAND1) and/or inhibiting the activity of the protein encoded by said gene, hereinafter also called "CAND1 inhibiting agent" is an agent capable of inhibiting the expression of CAND1. Illustrative non-limitative examples of such agents include small interfering RNAs (siRNAs), antisense oligonucleotides, morpholinos and small hairpin RNAs (shRNAs).

In another particular embodiment, the CAND1 inhibiting agent is a nucleic acid that specifically binds to the CAND1 gene or to the transcriptional product of said gene blocking the expression of said gene.

"Nucleic acids" as used herein mean biopolymers of nucleotides, which are linked with one another via phosphodiester bonds (polynucleotides or polynucleic acids). The nucleotides of a nucleic acid may additionally or alternatively be linked via phosphorothioate or phosphorodithionate bonds when chemically synthesized. Depending on the type of sugar in the nucleotides (ribose or deoxyribose), one distinguishes the two classes the ribonucleic acids (RNA) and the deoxyribonucleic acids (DNA). As used herein nucleic acid relates to any natural or non-natural nucleic acid and "oligonucleotide" and "polynucleotide" are used interchangeably in the context of the present invention. In the context of the present invention "Natural nucleotides" mean nucleotides that can be purified from natural sources. "Non-natural nucleotides" are defined as those produced using recombinant expression systems and, optionally, purified, chemically synthesized, etc. When appropriate, for example, in the case of chemically synthesized molecules, the nucleic acids can comprise nucleoside analogues such as analogues having chemically modified bases or sugars, modifications of the backbone, etc. A nucleic acid sequence is represented in 5'-3' direction unless indicated otherwise.

In a more particular embodiment the CAND1 inhibiting agent is a "small interfering RNA (siRNA). The term "siRNA" refers to a duplex of small inhibiting RNAs which induce the RNA interference pathway. Small interfering RNAs (siRNAs) are produced in cells by enzymatic cleavage of long dsRNAs by the RNase-III class endoribonuclease Dicer. The siRNAs associate with the RNA Induced Silencing Complex (RISC) in a process that is facilitated by Dicer. Dicer-Substrate RNAi methods take advantage of the link between Dicer and RISC loading that occurs when RNAs are processed by Dicer. Traditional 21-mer siRNAs are chemically synthesized RNA duplexes that mimic Dicer products and bypass the need for Dicer processing. Dicer-Substrate RNAs are chemically synthesized RNA duplexes that are optimized for Dicer processing. These molecules can vary in length (generally from 18 to 30 base pairs) and contain variable degrees of complementarity to their target mRNA in the antisense strand.

In an even more particular embodiment the inhibitor of CAND1 is a dicer-substrate 19-mer duplex siRNA molecule.

Some siRNAs, but not all, have unpaired overhanging bases at the 5' or 3' end of the sense strand and/or the antisense strand. In a particular embodiment the CAND1 inhibiting agent is a siRNA carrying one or more unpaired overhanging base(s) at the 3'end. In an even more particular embodiment the overhanging base(s) is/are thymine.

In a particular embodiment the CAND1 inhibiting siRNA displays two overhanging bases.

In a more particular embodiment the CAND1 inhibiting siRNA displays two overhanging thymines.

In a particular embodiment the CAND1-targeting siRNA binds to a polynucleotide sequence comprised within one or more than one of the mRNA molecules encoding the different CAND1 isoforms.

In a particular embodiment of the invention the CAND1 inhibiting siRNA comprises or consists of a region of the CAND1 mRNA. In a more particular embodiment of the invention the CAND1 inhibiting siRNA comprises or consists of the sequence 5'-UGGCCAAGAGCGAUAAUGG-3'. This sequence is referred to as the target region. This sequence corresponds to SEQ ID NO: 1.

In another particular embodiment the CAND1 inhibiting siRNA comprises or consists of the sequence 5'- UGGCCAAGAGCGAUAAUGGtt-3'. This sequence corresponds to SEQ ID NO: 2. This sequence is referred to as the sense strand.

In a more particular embodiment the CAND1 inhibiting siRNA comprises the sequence 5'- CCAUUAUCGCUCUUGGCCAtt-3'. This sequence corresponds to SEQ ID NO: 3. This sequence is referred to as the anti-sense strand.

In a particular embodiment of the invention the CAND1 inhibiting agent is a siRNA, wherein the siRNA has a sense strand defined by SEQ ID NO: 2 and an antisense strand defined by SEQ ID NO: 3.

As used herein the term "sense strand", also called "passenger" refers to the strand of the siRNA whose polynucleotide sequence is the same polynucleotide sequence comprised within the mRNA polynucleotide sequence it is targeting. As used herein the term "antisense strand", also called "guide" refers to the strand of the siRNA whose polynucleotide sequence is the complementary polynucleotide sequence to the polynucleotide sequence comprised within the mRNA it is targeting. As used herein the term "target region" refers to the polynucleotide sequence within a mRNA to which a siRNA binds.

Morpholinos are synthetic molecules which are the product of redesigning the natural nucleic acid structure. Structurally, the difference between morpholinos and DNA or RNA is that while morpholinos have standard nucleobases, those bases are linked to 6-membered morpholine rings instead of to deoxyribose/ribose rings, and the non-ionic phosphorodiamidate bonds between the subunits replace the anionic phosphodiester bonds. Morpholinos are sometimes referred to as PMO (phosphorodiamidate morpholino oligonucleotide). The 6 membered morpholine ring has the chemical formula O (CH2 CH2)2 NH. In this application the terms "phosphorodiamidate morpholine oligomer" and "morpholino are used interchangeably.

In a particular embodiment the CAND1 inhibiting agent is a phosphorodiamidate morpholine oligomer (PMO).

As it is used herein, the term "shRNA" or "short hairpin RNA" refers to a double stranded RNA (dsRNA) where the two strands are bound by a strand without interrupting the nucleotides between the 3' end of one strand and the 5' end of the other respective strand to form a duplex structure. shRNAs can be used to silence a target gene expression via RNA interference since, once the shRNA is processed it is located to the RNA-induced silencing complex (RISC), targeting RISC to an mRNA that has a complementary sequence. RISC may cleave this mRNA or repress its translation.

In a particular embodiment the CAND1 inhibiting agent is a short hairpin RNA (shRNA).

As previously mentioned, the mRNA molecules encoding the different CAND1 isoforms are defined by the sequences found in NCBI with the entry NM_001329674.2, NM_001329675.2, NM_001329676.2 and NM_018448.5.

In a particular embodiment the shRNA comprises a sequence ccomplementary to one or more than one of the mRNA molecules encoding the different CAND1 isoforms.

As it is used herein, an "antisense oligonucleotide" refers to an antisense polynucleotides comprising a single-stranded nucleic acid sequence (RNA or DNA) capable of binding to a target mRNA sequences (sense).

In a particular embodiment the CAND1 inhibiting agent is an antisense oligonucleotide.

In a more particular preferred embodiment the antisense oligonucleotide is an antisense RNA molecule.

In an even more particular embodiment the antisense oligonucleotide comprises a sequence ccomplementary to one or more than one of the mRNA molecules encoding the different CAND1 isoforms.

To evaluate whether an agent is capable of inhibiting the expression of Cullin Associated and Neddylation Dissociated 1 (*CAND1*), the mRNA levels of *CAND1* are determined. There are several techniques known to people skilled in the art to determine mRNA levels of CAND1 such as qPCR, Northern blot or RNA sequencing. Alternatively the protein levels of CAND1 can be used as a proxy to determine the mRNA levels of *CAND1.* Techniques to determine protein levels known to people skilled in the art comprise immunoblotting, mass spectrometry or immunohistochemistry.

In a particular embodiment the agent capable of inhibiting the expression of Cullin Associated and Neddylation Dissociated 1 (CAND1) and/or inhibiting the activity of the protein encoded by said gene, hereinafter also called "CAND1 inhibiting agent" is an agent capable of inhibiting the activity of CAND1.

In a particular embodiment the "*CAND1* inhibiting agent" is a "neutralizing antibody" or "an antigen-binding fragment thereof". As used herein, the term "antibody" refers to a protein including at least one immunoglobulin variable region, for example, an amino acid sequence providing an immunoglobulin variable domain or a sequence of the immunoglobulin variable domain. An "antigen-binding fragment" is any fragment of an antibody able to bind to CAND1and maintain its capacity to inhibit CAND1. An antibody can include, for example, a variable heavy chain (H) region (herein abbreviated as VH) and a variable light chain (L) region (herein abbreviated as VL). Typically, an antibody includes two variable heavy chain regions and two variable light chain regions. The term "antibody" encompasses antigen-binding antibody fragments (for example, single-chain antibodies, nanobodies (VHH), Fab fragments, F(ab')2 fragments, Fd fragments, Fv fragments and dAb fragments) as well as whole antibodies, for example, intact and/or full length immunoglobulins of the IgA, IgG types (for example, IgG1, IgG2, IgG3, IgG4), IgE, IgD, IgM (as well as subtypes thereof). The variable heavy and light chain regions can additionally be subdivided into hypervariability regions, referred to as "complementarity determining regions" ("CDR"), mixed together with more conserved regions, referred to as "framework regions" (FR). The extension of FRs and CDRs has been precisely defined (see Kabat, E.A., et al. (1991) Sequences of Proteins of Immunological Interest, Fifth Edition, The United States Department of Health and Human Services, NIH Publication No. 91-3242; and Chothia, C. etal. (1987) J. Mol. Biol. 196:901-917). Each variable heavy and light chain region is typically made up of three CDRs and four FRs, organized from the amino end to the carboxyl end in the following order: FR1, CDR1, FR2, CDR2, FR3, CDR3, FR4. The antibody VH or VL chain can furthermore include all or part of a heavy chain or light chain constant region to thereby form a heavy chain (HC) or light chain (LC) immunoglobulin, respectively. Immunoglobulin light and heavy chains can be bound by disulfide bridges. The heavy chain constant region typically includes three constant domains, CH1, CH2 and CH3. The light chain constant region typically includes a CL domain. The variable heavy and light chain region contains a binding domain interacting with an antigen. The constant regions of the antibodies typically mediate the binding of the antibody to host tissues or factors, including various cells of the immune system (for example, effector cells) and the first component (C1q) of the conventional complement system. The term antibody encompasses both antibodies formed by heavy chains and light chains and single-chain antibodies.

As it is used herein, the term "heavy chain" or "HC" encompasses both a full length heavy chain and fragments thereof. A full length heavy chain includes a variable region domain, VH, and three constant region domains, CH1, CH2 and CH3. The VH domain is at the amino terminal end of the polypeptide, and the CH3 domain is at the carboxyl terminal end.

As it is used herein, the term "light chain" encompasses a full length light chain and fragments thereof. A full length light chain includes a variable region domain, VL, and a constant region domain, CL. Like the heavy chain, the variable light chain region domain is at the amino terminal end of the polypeptide.

As it is used herein, the term "neutralizing", "neutralizing antibodies" or the like refer to an antibody or a functional fragment thereof binding to a target antigen of a protein and inhibiting or reducing the activity of said protein. The neutralizing ability of an antibody can be measured by several methods known to people skilled in the art. Said methods comprise virus neutralization assays, receptor-ligand binding assays or functional cellular assays.

In another particular embodiment the CAND1 inhibiting agent is a small molecule. The term "small molecule" refers to a low molecule weight (< 900 daltons) organic compound capable of regulating a biological process. Small molecules are capable of inhibiting a specific function of a protein or disrupting its protein-protein interactions.

In a preferred embodiment the small molecule destabilizes the interaction of CAND1 with Cullin1. In an even more preferred embodiment the small molecule is C60 as defined in PubChem CID 50759021.

Inhibition of the activity of a protein may be achieved by expressing another protein which regulates the function or structure or stability of the target protein. As it is used herein the term "stability" refers to the resistance of a polypeptide sequence of a protein to degradation. Degradation might occur as a result of the post translational modifications exerted by regulatory proteins onto the protein to be degraded. The term "posttranslational modification" refers to the covalent modification of a polypeptide sequence or a protein after its translation, generally through an isopeptide bond. Said modification is carried out by an enzyme. Posttranslational modifications comprise ubiquitination, NEDDylation and phosphorylation. Post translational modifications comprise the chemical modification of the target protein with one single chemical entity (i.e. one ubiquitin moiety, one NEDD8 moiety or one phosphate moiety) or with a polypeptide chain composed of one or more of the aforementioned chemical entities. Post translational modification also comprise the removal of already existing protein modifications (i.e. deubiquitination, deNEDDylation, dephosphorylation). As it is used herein the term "deNEDDylation" refers to the removal of the post translational modification NEDD8 attach to a target protein.

In a particular embodiment the CAND1 inhibiting agent is a polynucleotide sequence coding for a protein that regulates the stability of CAND1

In a more particular embodiment the CAND1 inhibiting agent exerts its function by removing the post translational modification known as NEDD8.

In an even more particular embodiment the CAND1 inhibiting agent is NEDD8-specific Protease 1 (NEDP1).

In a yet even more particular embodiment the CAND1 inhibiting agent is a polynucleotide sequence coding for NEDD8-specific Protease 1 (NEDP1).

The expression of NEDP1 encoded by the aforementioned polynucleotide sequence requires the delivery of said polynucleotide sequence into a host cell. There are several modes of delivery known to people skilled in the art. Suitable delivery methods may depend on the size of the polynucleotide sequence or the target cells. In a particular embodiment the mode of delivery is virus based, preferably based on lentivirus or Adenovirus (AAV). In a particular embodiment the polynucleotide sequence coding for NEDP1 further comprises the sequence of a promoter. In some even more particular embodiments said promoter is a constitutive promoter, preferably a CMV promoter. In some other even more particular embodiments said promoter is a tissue specific promoter, preferably liver specific promoter.

In a particular embodiment the polynucleotide sequence coding for NEDP1 is comprised as part of a plasmid. In a more particular embodiment said plasmid is a transfer plasmid. In a yet more particular embodiment said transfer plasmid is a lentivirus transfer plasmid. In another yet more particular embodiment said packaging plasmid is a AAV transfer plasmid.

In some particular embodiments a packaging plasmid and/or an envelope plasmids are additionally used to generate suitable lentivirus or adenovirus. In some particular embodiments packaging cells are transfected with the transfer plasmid, packaging plasmid and/or envelop plasmid to generate the viruses. In a particular embodiment the Adenoviruses have a tropism for liver cells. In a more particular embodiment, the serotype of the adenovirus is AA7, AA8 or AAV9.

The term "NEDP1" refers to the protein coded by the gene *SENP8.* NEDD8-specific protease 1 or NEDP1 has protease activity and processes the full length NEDD8 to its mature form and deconjugates NEDD8 from target proteins. The human NEDP1 corresponds to the gene *SENP8* identified in the NCBI database by the gene ID 123228 (release date 8 February 2025)

The protein encoded by *SENP8* is called NEDD8-specific protease 1 or "NEDP1". In a particular embodiment NEDP1 is human NEDP1 as defined in the UniProt database with the accession number Q96LD8, entry version 177, sequence version 1, release date 5 February 2025.

As previously described, siRNAs may be employed to inhibit the expression of a target mRNA molecule. Methods for in vivo delivery of siRNAs are known in the art (Davis, M. E. et al., Nature 2010 April 15; 464(7291): 1067-1070).

The chemical conjugation of specific moieties to siRNAs confers them specificity (i.e. cell type specificity). The ligand is preferably specific for a receptor expressed on the surface of a target cell. For siRNAs that are to be targeted to hepatocytes, the ligand is preferably a GalNAc or a GalNAc derivative.

In a particular embodiment, the siRNA is conjugated to at least one ligand.

In a more particular embodiment the siRNA is conjugated to at least one N-acetylgalactosamine (GalNAC).

In an even more particular embodiment the siRNA is conjugated to a triantennary N-acetylgalactosamine (GalNAC).

The nucleic acids of the invention may be conjugated to a ligand. Efficient delivery of oligonucleotides, in particular double-stranded nucleic acids of the invention, to cells in vivo is important and requires specific targeting and substantial protection from the extracellular environment, particularly serum proteins. One method of achieving specific targeting is to conjugate a ligand to the nucleic acid. In some embodiments, the ligand helps in targeting the nucleic acid to a target cell which has a cell surface receptor that binds to and internalizes the conjugated ligand. In such embodiments, there is a need to conjugate appropriate ligands for the desired receptor molecules in order for the conjugated molecules to be taken up by the target cells by mechanisms such as different receptor-mediated endocytosis pathways or functionally analogous processes. In other embodiments, a ligand which can mediate internalization of the nucleic acid into a target cell by mechanisms other than receptor mediated endocytosis may alternatively be conjugated to a nucleic acid of the invention for cell or tissue specific targeting.

One example of a conjugate that mediates receptor mediated endocytosis is the asialoglycoprotein receptor complex (ASGP-R) which has high affinity to the GalNAc moiety described herein. The ASGP-R complex is composed of varying ratios of multimers of membrane ASGR1 and ASGR2 receptors, which are highly abundant on hepatocytes. One of the first disclosures of the use of triantennary cluster glycosides as conjugated ligands was in US patent number US 5,885,968. Conjugates having three GalNAc ligands and comprising phosphate groups are known and are described in Dubber et al. (Bioconjug. Chem. 2003 Jan-Feb;14(1):239-46.). The ASGP-R complex shows a 50-fold higher affinity for N-Acetyl-D-Galactosamine (GalNAc) than D-Gal.

The ASGP-R complex recognizes specifically terminal β-galactosyl subunits of glycosylated proteins or other oligosaccharides (Weigel, P.H. et. al., Biochim. Biophys. Acta. 2002 Sep 19;1572(2-3):341-63) and can be used for delivering a drug to the liver's hepatocytes expressing the receptor complex by covalent coupling of galactose or galactosamine to the drug substance (Ishibashi,S.; et. al., J Biol. Chem. 1994 Nov 11;269(45):27803-6). Furthermore, the binding affinity can be significantly increased by the multi-valency effect, which is achieved by the repetition of the targeting moiety (Biessen EA, et al., J Med Chem. 1995 Apr 28;38(9):1538-46).

The ASGP-R complex is a mediator for an active uptake of terminal β-galactosyl containing glycoproteins to the cell's endosomes. Thus, the ASGPR is highly suitable for targeted delivery of drug candidates conjugated to such ligands like, e.g., nucleic acids into receptor-expressing cells (Akinc et al., Mol Ther. 2010 Jul;18(7):1357-64). More generally the ligand can comprise a saccharide that is selected to have an affinity for at least one type of receptor on a target cell. In particular, the receptor is on the surface of a mammalian liver cell, for example, the hepatic asialoglycoprotein receptor complex described before (ASGP-R).
The saccharide may be selected from N-acetyl galactosamine, mannose, galactose, glucose, glucosamine and fucose. The saccharide may be N-acetyl galactosamine (GalNAc).

A ligand for use in the present invention may therefore comprise (i) one or more N-acetyl galactosamine (GalNAc) moieties and derivatives thereof, and (ii) a linker, wherein the linker conjugates the GalNAc moieties to a nucleic acid as defined in any preceding aspects. The linker may be a monovalent structure or bivalent or trivalent or tetravalent branched structure. The nucleotides may be modified as defined herein. In some embodiments the siRNA is modified with at least one of the modifications selected from modifications of the sugar at 2' such as 2'-O-methyl (2'-OMe), 2'-O-methoxyethyl (2'-MOE), 2'-O- methoxyethoxy, 2'-fluoro (2'-F), 2'-allyl, 2'-O-[2-(methylamino)-2-oxoethyl], 2'-O-(N-methylcarbamate); modifications of the sugar at 4' including 4'-thio, 4'-CH2-O-2' bridge, 4-(CH2)2-O-2' bridge; locked nucleic acid (LNA); peptide nucleic acid (PNA); intercalating nucleic acid (INA); twisted intercalating nucleic acid (TINA); hexitol nucleic acids (HNA); arabinonucleic acid (ANA); cyclohexene nucleic acids (CNAs); cyclohexenyl nucleic acid (CeNA); threose nucleic acid (TNA); morpholine oligonucleotides; Gapmers; Mixmers; incorporation of arginine-rich peptides; addition of 5'-phosphate to synthetic RNAs; RNA aptamers (Que-Gewirth NS, Gene Ther. 2007 Feb;14(4):283-91.); antidote-controlled RNA aptamers in the subject of the specific RNA aptamer (ref. Oney S, Oligonucleotides. 2007 Fall; 17(3):265-74), or any combination thereof.

In some particular embodiments the chemical modifications are 2-O-methyl and/or 2'deoxy-2'fluoryl.

In some particular embodiments the chemical modifications are vinyl phosphonate.

In some embodiments one or more nucleosides bonds of the siRNA is modified with at least one of the modifications selected from: phosphorothioate, phosphorodithioate, phosphoramidate, phosphorodiamidate, phosphorodithioate, phosphoroselenoate, phosphorodiselenoate, phosphoroanilothioate, and phosphoroanilidate, or any combination thereof, preferably a phosphorothioate

As previously mentiond the ligand may therefore comprise GalNAc. In a particular embodiment the ligand is a triantennary glycoside, preferably N-acetylgalactosamine (GalNAC), more preferably the triannternary GalNAC with the following structure: 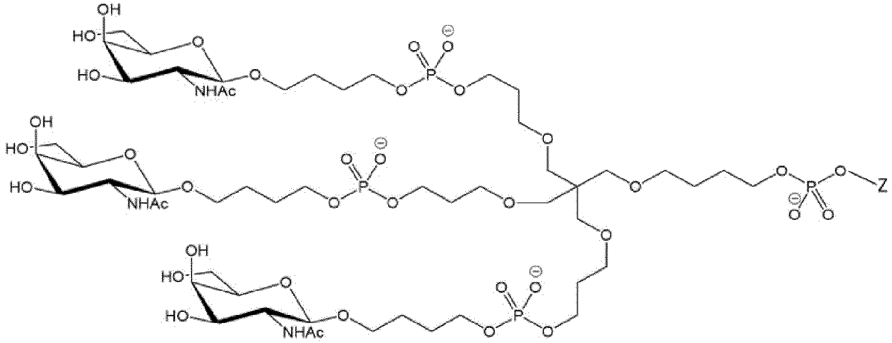 wherein Z is any nucleic acid as disclosed herein.

In some embodiments the triantennary N-acetylgalactosamine (GalNAC) moiety is conjugated to the 3'end or the 5'end of the sense strand, preferably the 5'end.

In some embodiments the triantennary N-acetylgalactosamine (GalNAC) moiety is conjugated to the sense strand or the antisense strand, preferably the sense strand.

In a more particular embodiment the triantennary N-acetylgalactosamine (GaINAC) moiety is conjugated to the siRNA molecule via means of a thiol-maleimide reaction click chemistry, amine-carboxylic acid reaction, or thiol-ene reaction.

In another more particular embodiment the siRNA is conjugated to at least one ligand comprising a compound formula defined in (1)

(1) S-X1-P-X2]₃-A-X3-

Wherein:
- S is the triantennary N-acetylgalactosamine
- X1 is a C4 alyklene
- P is a phosphate or modified phosphate which is phosphothioate
- X2 is (-CH₂)n-O-CH₂- where n = 3
- A is a branching unit corresponding to the compound defined in formula (II): 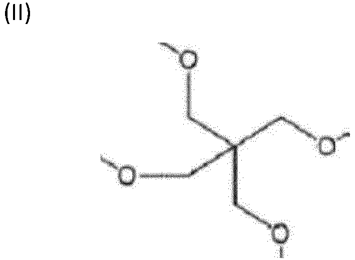
- X3 represents a bridging unit, for example -CH2)n-O-CH2-

The nucleic acid of the siRNA is conjugated to X3 via a phosphate or modified phosphate which is a phosphothioate.

In a particular embodiment, siRNA is used at a concentration of between 0.1µg/µl and 2,5 µg/ml. In a preferred embodiment, siRNA is used at a concentration of at least 0.1µg/µl, at least 0.2µg/µl, at least 0.3µg/µl, at least 0.4µg/µl, at least 0.5µg/µl, at least 0.6µg/µl, at least 0.7µg/µl, at least 0.8µg/µl, at least 0.9µg/µl, at least 1 µg/µl, at least, 1.1 µg/µl, at least 1.2µg/µl, at least 1.3µg/µl, at least 1.4µg/µl, at least 1.5µg/µl, at least 1.6µg/µl, at least 1.7µg/µl, at least 1.8µg/µl, at least 1.9µg/µl, at least 2 µg/µl, least 2.1µg/µl, at least 2.2µg/µl, at least 2.3µg/µl, at least 2.4µg/µl, at least 2.5 µg/µl, or more. In another preferred embodiment, siRNA is used at a concentration of 0.75 µg/µl.

In a particular embodiment, siRNA is used at a dose of between 0.1 and 10 mg per kilogram of patient. In a preferred embodiment, siRNA is used at a dose of at least 0.1 mg/kg, at least 0.5 mg/kg, at least 1 mg/kg, at least 5 mg/kg, at least 10 mg/kg, at least 20 mg/kg, at least 30 mg/kg.

In a preferred embodiment, the CAND1 inhibiting agent is administered once a day, twice a day, thrice a day, or more. In another preferred embodiment, the CNNM4 inhibitor is administered once a week, twice a week, 3 times a week, 4 times a week, 5 times a week, 6 times a week, 7 times a week, or more. In a more preferred embodiment, the CAND1 inhibiting agent is administered twice a week.

Some tumors display high levels of post translational modifications which promote cell growth or inhibit cell death (Zhould et al., 2019; Geffen et al., 2023). One such type of post translational modification is NEDDylation.

In a particular embodiment the CAND1 inhibiting agent is used for the treatment and/or prevention of HB or HCC tumors characterized by having high levels of NEDDylation. Alternatively, the CAND1 inhibiting agent of the invention is used for the treatment and/or prevention of any tumor characterized by having high levels of NEDDylation.

As it is used herein the term "high levels of NEDDylation" refers to higher expression of NEDD8 and NEDDylation enzymes such as NAE1/UBA3 and UBC12 in the cancer tissue compared with a reference value.

The term "reference value", as used herein, relates to a predetermined criteria used as a reference for evaluating the values or data obtained from the samples collected from a subject. The reference value or reference level can be an absolute value, a relative value, a value that has an upper or a lower limit, a range of values, an average value, a median value, a mean value, or a value as compared to a particular control or baseline value. A reference value can be based on a large number of samples, such as from population of subjects of the chronological age matched group, or based on a pool of samples. As used herein the term "reference value" refers to the expression level values of any gene product (i.e. NEDP1 mRNA transcript, NEDP1 protein, CAND1 mRNA transcript or CAND1 protein) determined from the tissue of non-cancer sample controls or subjects. The expression of NEDD8 or the NEDDylation enzymes is evaluated based on the transcript levels and/or protein levels by using techniques know to people skilled in the art (i.e. qPCR, immunohistochemistry, immunoblotting or mass spectrometry).

The reference value can also be based on an individual sample value, such as for example, a value obtained from a sample from the subject being tested, but at an earlier point in time or from a non-cancerous tissue. The reference value can be based on a large number of samples, such as from population of subjects of the chronological age matched group, or based on a pool of samples including or excluding the sample to be tested. Various considerations are taken into account when determining the reference value of the marker. Among such considerations are the age, weight, sex, general physical condition of the patient and the like. For example, equal amounts of a group of at least 2, at least 10, at least 100 to preferably more than 1000 subjects, preferably classified according to the foregoing considerations, for example according to various age categories, are taken as the reference group.

In some embodiments the expression levels are evaluated as a function of the mRNA levels or protein levels of NEDD8 or the NEDDylation enzymes with respect to a housekeeping control.

The term "house-keeping control" refers to a gene whose mRNA transcript levels or protein levels remain unchanged in tumor and non- tumor samples.

In particular embodiments the house keeping control mRNA transcript or protein is HSP90. In other particular embodiments the house keeping control mRNA transcript or protein is β-actin.

A tumor is considered to display high levels of NEDDylation with respect to the reference value when the mRNA transcript levels of the NEDDylation enzymes, comprising NAE1, Cbl or Uchl1, are increased by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95% or by at least 99%.

A tumor is considered to display high levels of NEDDylation with respect to the reference value when the protein levels of the NEDDYLATION enzymes, comprising NAE1, Cbl, Uchl1, CAND1 or the protein levels of NEDD8 are increased by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95% or by at least 99%.

A tumor is considered to display high levels of NEDDylation with respect to the reference value when the mRNA and/or protein levels of NEDP1 are decreased by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95% or by at least 99%.

### Diagnostic method of the invention

In a second aspect the present invention relates to an *in vitro* method for the diagnosis of a hepatoblastoma or a hepatocellular carcinoma in a subject, the method comprising:
1. determining *CAND1* and/or *NEDP1* expression levels in a liver biopsy sample from a subject and,
2. comparing these levels with a reference value,
wherein increased expression levels of *CAND1* and/or decreased expression levels of *NEDP1* compared with the reference value are indicative that the subject suffers from a hepatoblastoma or a hepatocellular carcinoma.

The terms "hepatoblastoma", "hepatocellular carcinoma", "CAND1", "NEDP1" and "subject" have been previously described in connection with the first aspect of the invention. All the particular and prefer embodiments of these terms also apply to the second aspect of the invention.

The term *"in vitro",* as used herein, refers to processes or reactions conducted outside an organism in conditions resembling or in the exact same conditions as those found within said organism.

As used herein the term "diagnosis" refers to the process of attempting to determine and/or identify a possible disease in a subject, i.e. the diagnostic procedure, and to the opinion reached by this process, i.e. the diagnostic opinion. As such, it can also be regarded as an attempt at classification of an individual's condition into separate and distinct categories that allow medical decisions about treatment to be made. As the person skilled in the art will understand, such a diagnosis, it may not be correct for 100% of the subjects. The diagnosis, however, requires that a statistically significant part of the subjects can be identified as suffering from a disease, particularly a liver disease, more particularly liver cancer and even more particularly hepatoblastoma or hepatocellular carcinoma. The skilled in the art may determine whether a party is statistically significant using different statistical evaluation tools well known to people skilled in the art, for example, by determination of confidence intervals, the p-value determination, Student's-test, the Mann- Whitney, etc. (see Dowdy and Wearden, 1983). Preferred confidence intervals are at least, 50%, at least 60%, at least 70%>, at least 80%>, at least 90%) or at least 95%. The p-values are preferably, 0.05, 0.025, 0.001 or lower. The term "diagnosing" as disclosed herein means the process of determining which disease or condition explains a person's symptoms and signs.

As used herein the term "biopsy" refers to a medical produced wherein a small sample of tissue is removed from the body for further examination for the diagnosis of a disease.

In some embodiments the tissue used for the diagnosis a hepatoblastoma or hepatocellular carcinoma is a biopsy tissue.

In some other particular embodiments the tissue used for the diagnosis of a hepatoblastoma or hepatocellular carcinoma is a liver biopsy tissue.

In some other more particular embodiments the diagnosis of a hepatoblastoma or hepatocellular carcinoma is determined by measuring the expression levels *CAND1* and/or *NEDP1* in a liver biopsy tissue.

In some other even more particular embodiments the diagnosis of a hepatoblastoma or hepatocellular carcinoma is determined by measuring the *CAND1* and/or *NEDP1* mRNA transcript levels in a liver biopsy tissue.

The methods suitable for determining the mRNA transcript levels of CAND1 and/or NEDP1 are those common to people skilled in the art, i.e. qPCR, RT-PCR, Northern blot, RNA dot blot, in situ hybridization, microarray technology, tag based methods such as serial analysis of gene expression (SAGE) including variants such as LongSAGE and SuperSAGE, microarrays, fluorescence in situ hybridization (FISH), including variants such as Flow-FISH, qFiSH and double fusion FISH (D-FISH), RNA sequencing and the like.

In some embodiments the mRNA transcript levels are expressed as relative values with respect to a house keeping gene, preferably *HSP90* or β-*Actin.*

In a particular embodiment the mRNA transcript levels are determined by measuring mRNA transcript levels using qPCR.

In a particular embodiment the mRNA transcript levels are determined by measuring mRNA transcript levels using RNA seq.

In some embodiments the method for diagnosing hepatoblastoma or hepatocellular carcinoma of the invention comprises determining the mRNA transcript level of *CAND1.*

In a particular embodiment the method for diagnosing hepatoblastoma or hepatocellular carcinoma of the invention comprises determining the mRNA transcript levels of *CAND1* by qPCR.

In a particular embodiment the method for diagnosing hepatoblastoma or hepatocellular carcinoma of the invention comprises determining the mRNA transcript levels of *CAND1* by RNA seq.

In a particular embodiment the method for diagnosing hepatoblastoma or hepatocellular carcinoma of the invention comprises determining the mRNA transcript levels of *CAND1* by Human Transcriptome Array (HTA).

In some embodiments the method for diagnosing hepatoblastoma or hepatocellular carcinoma of the invention comprises determining the mRNA transcript level of *SENP8.*

In a particular embodiment the method for diagnosing hepatoblastoma or hepatocellular carcinoma of the invention comprises determining the mRNA transcript levels of *SENP8* by qPCR.

In a particular embodiment the method for diagnosing hepatoblastoma or hepatocellular carcinoma of the invention comprises determining the mRNA transcript levels of *SENP8* by RNA seq.

In a particular embodiment the method for diagnosing hepatoblastoma or hepatocellular carcinoma of the invention comprises determining the mRNA transcript levels of *SENP8* by Human Transcriptome Array (HTA).

In some embodiments the method for diagnosing hepatoblastoma or hepatocellular carcinoma of the invention comprises determining the expression level of CAND1 and/or NEDP1 protein.

In some embodiments the expression levels of *CAND1* and/or *NEDP1* from a liver biopsy sample from a subject are determined by measuring protein levels. Methods suitable for determining the expression level of CAND1 and/or NEDP1 protein include, without limitation, comprise quantification by means of conventional methods, for example, using antibodies capable of binding specifically to CAND1 and/or NEDP1 protein and the subsequent quantification of the resulting antibody-antigen complexes. There is a wide range of well-known assays which can be used in the present invention, using non-labelled antibodies (primary antibody) and labelled antibodies (secondary antibody); these techniques include, among others, Western blot, ELISA (enzyme-linked immunosorbent assay), RIA (radioimmunoassay), competitive EIA (competitive enzyme immunoassay), DAS-ELISA (double antibody sandwich ELISA), immunocytochemical and immunohistochemical techniques, techniques based on the use of biochips or protein microarrays including specific antibodies, or assays based on colloidal precipitation in formats such as dipsticks. Other ways of detecting and quantifying the levels of the protein of interest include affinity chromatography techniques, mass spectrometry, ligand binding assays, and the like.

In a preferred embodiment the method for diagnosing hepatoblastoma or hepatocellular carcinoma comprises determining the expression levels of CAND1 and/or NEDP1 protein by immunohistochemistry.

In another preferred embodiment the method for diagnosing hepatoblastoma or hepatocellular carcinoma comprises determining the expression levels of CAND1 and/or NEDP1 protein by immunoblotting.

In another preferred embodiment the method for diagnosing hepatoblastoma or hepatocellular carcinoma comprises determining the expression levels of CAND1 and/or NEDP1 protein by mass spectrometry.

In the context of the method to determine whether a subject suffers from liver cancer, more specifically hepatoblastoma or hepatocellular carcinoma, the expression levels of CAND1 and/ or NEDP1 are compared to a reference value.

The term "reference value" has been previously defined.

In a particular embodiment, when the *CAND1* or *NEDP1* expression levels are determined as mRNA transcript levels, the term "reference value" refers to the mRNA transcript levels of *CAND1* and/or *NEDP1* in the tissue of liver biopsies from healthy subjects. In a more particular embodiment the mRNA transcript levels of *CAND1* and/or *NEDP1* in the tissue from liver biopsies of healthy subjects are determined using a Human Transcriptome Array (HTA).

The reference values of CAND1 and/or NEPD1 transcript levels in the tissue from liver biopsies of healthy subjects is defined as the average of the transcript levels of CAND1 from the liver biopsies of at least one healthy subjects.

In a particular embodiment the reference values of CAND1 transcript levels in the tissue from liver biopsies of healthy subjects is defined as the average of the transcript levels of CAND1 from the liver biopsies of 20 healthy subjects.

The skilled person in the art may determine whether the expression levels of CAND1 and/or NEDP1 from a liver biopsy sample are significantly different from a reference value. To this end, different statistical evaluation tools well known to people skilled in the art are used. For example, by determination of confidence intervals, the p-value determination, Student's-test, the Mann- Whitney, etc. (see Dowdy and Wearden, 1983). Preferred confidence intervals are at least, 50%, at least 60%, at least 70%>, at least 80%>, at least 90%) or at least 95%. The p-values are preferably, 0.05, 0.025, 0.001 or lower. These p-values are deemed significant therefore indicating that the difference is statistically significant.

In a particular embodiment if the p-value of the comparison between the reference value and the value obtained from the liver biopsy sample of the subject is deemed significant, the patient is diagnosed as suffering from a liver cancer, more specifically hepatoblastoma or hepatocellular carcinoma.

In a particular embodiment, the expression of a *CAND1* is considered increased in a sample of the patient under study when the levels increase with respect to the reference values by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%, by at least 110%, by at least 120%, by at least 130%, by at least 140%, by at least 150%, or more. Similarly, the expression of a NEDP1 is considered decreased when its levels decrease with respect to the reference sample by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100% (i.e., absent).

In another particular embodiment the term "reference value" refers to the protein levels of CAND1 and/or NEDP1 in the tissue of liver biopsies from healthy subjects. In a more particular embodiment the protein levels of CAND1 and/or NEDP1 in the tissue from liver biopsies of healthy subjects are determined using immunoblotting. In an even more particular embodiment the protein levels of CAND1 and/or NEDP1 in the tissue from liver biopsies of health subjects are represented as the fold change of the band intensity of CAND1 and/or NEDP1 with respect to the band intensity of a house keeping control, preferably β-Tubulin.

In a particular embodiment the "reference values" is understood as the fold change of the band intensity of CAND1 and/or NEDP1 with respect to the band intensity of a house keeping control, preferably β-Tubulin and this value is set to 1.

In a particular embodiment the value corresponding to the sample from the subject suspected to have hepatoblastoma or hepatocellular carcinoma is determined as the band intensity of CAND1 and/or NEDP1 with respect to the band intensity of a house keeping gene, preferably β-Tubulin.

In another particular embodiment said value is normalized to the "reference value" described above.

In a particular embodiment the value of the subject and the reference value are compared to determine whether there is a significant difference.

The skilled person in the art may determine whether the relative protein levels of CAND1 and/or NEDP1 from a liver biopsy sample are significantly different from a reference value. To this end, different statistical evaluation tools well known to people skilled in the art are used. For example, by determination of confidence intervals, the p-value determination, Student's-test, the Mann- Whitney, etc. (see Dowdy and Wearden, 1983). Preferred confidence intervals are at least, 50%, at least 60%, at least 70%>, at least 80%>, at least 90%) or at least 95%. The p-values are preferably, 0.05, 0.025, 0.001 or lower. Any p-value equal or lower than 0.05 are deemed significant, thereby indicating that the difference is statistically significant.

In a particular embodiment when the p-value of the comparison between the reference value and the value obtained from the liver biopsy sample of the subject suspected to have hepatoblastoma or hepatocellular carcinoma, is deemed significant, the patient is diagnosed as suffering from a liver cancer, more specifically hepatoblastoma or hepatocellular carcinoma.

In an alternative aspect of this invention when the expression levels of CAND1 are increased and/or the expression levels of NEDP1 are decreased compared with the reference value the patient suffers from a liver cancer.

In other embodiment when the expression levels of CAND1 are increased and/or the expression levels of NEDP1 are decreased compared with the reference value the patient suffers from hepatoblastoma.

In yet another embodiment when the expression levels of CAND1 are increased and/or the expression levels of NEDP1 are decreased compared with the reference value the patient suffers from hepatocellular carcinoma.

### Prognosis method of the invention.

In a third aspect, the present invention relates to an *in vitro* method for the determining the prognosis of a patient with a hepatoblastoma or hepatocellular carcinoma, the method comprising:
(i) determining *CAND1* and/or *NEDP1* expression levels in a liver biopsy sample from a subject and,
(ii) comparing these levels with a reference value,
wherein:
- if the expression levels of *CAND1* are increased compared to a reference value and/or,
- if the expression levels of *NEDP1* are decreased compared to a reference value the patient has a poor prognosis.

The terms "hepatoblastoma, "hepatocellular carcinoma", "CAND1", "NEDP1", "biopsy", "subject", *"in vitro"* have been previously described in connection with the first and/or second aspects of the invention. All the particular and prefer embodiments of these terms also apply to the third aspect of the invention.As used herein the term "prognosis" refers to the process of attempting to determine the future state of a patient suffering from a particular disease or condition. As such, it can be regarded as an attempt at classifying the likely outcome of a disease of a patient into separate and distinct categories that informs future medical decisions. As the person skilled in the art will understand, such a prognosis might not be correct for 100% of the subjects, although such an outcome would be preferred. The term, however, requires that a statistically significant part of the subjects can be identified as having a poor prognosis. The skilled person in the art may determine whether a party is statistically significant using the different statistical evaluation tools previously described.

As used herein, "poor prognosis" indicates that the patient is expected, i.e. predicted, to not survive and/or to have, or is at high risk of having, recurrence or distant metastases within a set time period. The term "high" is a relative term and, in the context of this invention, refers to the risk of the "high" expression group with respect to a clinical outcome (recurrence, distant metastases, etc.). A "high" risk can be considered as a risk higher than the average risk for a heterogeneous cancer patient population. The risk will also vary in function of the time period. The time period can be, for example, five years, ten years, fifteen years or even twenty years of initial diagnosis of cancer or after the prognosis was made.

In a particular embodiment, poor prognosis refers to low overall survival and/or low event-free survival.

In some embodiments the expression levels of *CAND1* and/or *NEDP1* from a liver biopsy sample from a subject are determined by measuring mRNA transcript levels. The methods suitable for determining the mRNA transcript levels of CAND1 and/or NEDP1 are those common to people skilled in the art, i.e. qPCR, RT-PCR, Northern blot, RNA dot blot, in situ hybridization, microarray technology, tag based methods such as serial analysis of gene expression (SAGE) including variants such as LongSAGE and SuperSAGE, microarrays, fluorescence in situ hybridization (FISH), including variants such as Flow-FISH, qFiSH and double fusion FISH (D-FISH), RNA sequencing and the like. In some embodiments the mRNA transcript levels are expressed as relative values with respect to a house keeping gene, preferably β-actin or HSP90. In a preferred embodiment the mRNA transcript levels are determined by measuring mRNA transcript levels using qPCR. In a preferred embodiment the mRNA transcript levels are determined by measuring mRNA transcript levels using RNA seq. In another preferred embodiment the *CAND1* mRNA transcript levels are determined using Human Transcriptome Array (HTA).

In some embodiments the method for determining the prognosis of a patient with hepatoblastoma or hepatocellular carcinoma of the invention comprises determining the mRNA transcript level of CAND1.

In a preferred embodiment the method for determining the prognosis of a patient with hepatoblastoma or hepatocellular carcinoma of the invention comprises determining the mRNA transcript levels of CAND1 by qPCR.

In a preferred embodiment the method for determining the prognosis of a patient with hepatoblastoma or hepatocellular carcinoma of the invention comprises determining the mRNA transcript levels of CAND1 by RNA seq.

In a preferred embodiment the method for determining the prognosis of a patient with hepatoblastoma or hepatocellular carcinoma of the invention comprises determining the mRNA transcript levels of CAND1 by Human Transcriptome Array (HTA).

In the context of the method to determine the prognosis of a patient with hepatoblastoma or hepatocellular carcinoma, the expression levels of CAND1 are compared to a reference value, so as if the expression levels of CAND1 are increased with respect to a reference value and or the expression levels of NEDP1 are decreased compared to a reference value, the patient has poor prognosis. The methods for determining CAND1 and NEDP1 expression levels, either based on mRNA transcript levels or in the protein levels, as well as reference levels previously explained for the second method of the invention also apply to the third method of the invention.

In a particular embodiment, to determine the prognosis of a patient with hepatoblastoma, the CAND1 mRNA transcript levels of a liver biopsy sample of a patient with hepatoblastoma is compared to the CAND1 mRNA transcript levels of a database of hepatoblastoma. Said database stratifies subjects suffering from hepatoblastoma on the basis of CAND1 mRNA transcript levels. Said database correlates the CAND1 mRNA transcript levels of each patient to their overall or event free survival. As such, subjects who display a CAND1 mRNA transcript level above the 75^{th} percentile display an average 50% event free survival. Subjects who display a CAND1 mRNA transcript level below the 75^{th} percentile display an average 70.8% event free survival. Similarly, subjects who display a CAND1 mRNA transcript level above the 75^{th} percentile display an average 50% overall survival. Subjects who display a CAND1 mRNA transcript level below the 75^{th} percentile display an average 83.3% overall survival.

In the context of this invention the term "overall survival" refers to the survival of a patient suffering from a disease, specifically liver cancer and more specifically hepatoblastoma, irrespective of whether or not said patient has any other additional tumor(s) or metastasis of the original tumor.

In the context of this invention the term "event free survival" refers to the survival of a patient suffering from a disease, specifically liver cancer and more specifically hepatoblastoma, wherein said patient has other additional tumor(s) or metastasis of the original tumor.

In a particular embodiment when the expression levels of CAND1 mRNA transcript in a patient suffering from a hepatoblastoma is a value above the value corresponding to the 75^{th} percentile the event-free survival is 50%.

In a particular embodiment when the expression levels of CAND1 mRNA transcript in a patient suffering from a hepatoblastoma is a value below the value corresponding to the 75^{th} percentile, the event-free survival is 70.8%

In a particular embodiment when the expression levels of CAND1 mRNA transcript in a patient suffering from a hepatoblastoma is a value above the value corresponding to the 75^{th} percentile the overall survival is 50%.

In a particular embodiment when the expression levels of CAND1 mRNA transcript in a patient suffering from a hepatoblastoma is a value below the value corresponding to the 75^{th} percentile, the overall survival is 83.3%

In a particular embodiment when the overall survival or the event free survival is 50% or lower the prognosis of a patient suffering from hepatoblastoma is understood as poor.

In a particular embodiment when the overall survival or the event free survival is higher than 50%, preferably higher than 83.3% or 70.8% respectively the prognosis of a patient suffering from hepatoblastoma is understood as good.

In a particular embodiment the prognosis of a patient suffering from hepatocellular carcinoma is determined by measuring CAND1 expression levels in the tumor

In an even more particular embodiment the prognosis of a patient suffering from hepatocellular carcinoma is determined based on the CAND1 mRNA transcript levels.

The methods to measure the mRNA transcript levels in the liver biopsies has been have been previously described in connection with other aspects of the invention. All the particular and preferred embodiments of the other aspects of the invention regarding these terms fully apply to this aspect.

In a particular embodiment to determine the prognosis of a patient suffering from hepatocellular carcinoma, the CAND1 mRNA transcript levels of a liver biopsy sample of a patient suffering from a hepatocellular carcinoma is compared to the CAND1 mRNA transcript levels of a database of patients suffering from hepatocellular carcinoma. Said database stratifies subjects suffering from hepatocellular carcinoma on the basis of CAND1 mRNA transcript levels. Said database correlates the CAND1 mRNA transcript levels of each patient to their survival. Patients suffering from hepatocellular carcinoma were stratified into CAND1 high expression group (values higher than 15.38 Transcripts per Million Reads) and CAND1 low expression group (values higher than 15.38 Transcript per Million Reads). Patients classified as displaying high expression of CAND1 have on average a 33% chance or lower of survival while patients classified as displaying low expression of CAND1 have on average a 53% chance or higher of survival.

In a particular embodiment a patient suffering from hepatocellular carcinoma and displaying a CAND1 mRNA transcript level higher than 15.38 Transcripts per Million Reads is understood to have a high expression of CAND1.

In a particular embodiment a patient suffering from hepatocellular carcinoma and displaying a CAND1 mRNA transcript level lower than 15.38 Transcripts per Million Reads is understood to have a low expression of CAND1.

In a particular embodiment the term "good prognosis" is understood as having a 53% chance or higher of survival.

In a particular embodiment the term "poor prognosis is understood as having a 55% or lower chance of survival, particularity a 33% chance or lower of survival.

In a particular embodiment a patient suffering from hepatocellular carcinoma and displaying an increased expression levels of CAND1 compared to a reference value has a poor prognosis.

In a particular embodiment a patient suffering from hepatocellular carcinoma and displaying decreased expression levels of CAND1 compared to a reference value has a good prognosis.

### Method for determining the predisposition of a subject to developing hepatoblastoma or hepatocellular carcinoma

In a fourth aspect, the invention relates to an in vitro method for determining the predisposition of a subject to developing hepatoblastoma or hepatocellular carcinoma, the method comprising:
(i) determining the deNEDDylation activity in a liver biopsy sample and
(ii) comparing these levels with a reference value
wherein the decreased levels of deneddylation activity of the sample compared to a reference value are indicative of increased chances of developing a hepatoblastoma or a hepatocellular carcinoma.

The terms "hepatoblastoma, "hepatocellular carcinoma", "CAND1", "NEDP1", "biopsy", "subject" and *"in vitro"* have been previously described in connection with the previous aspects. All the particular and preferred embodiments of these terms also apply to the fourth aspect of the invention.

The term "predisposition" refers to the increased likelihood of a subject to developing a particular disease. Such predisposition may be assessed using medical history, genomic analysis or cellular assays.

The first step of the method of the fourth aspect comprises determining the deNEDDylation activity in a liver biopsy sample from the subject. In a particular embodiment, this is determined using a cellular assay.

In an even more particular embodiment the cellular assay employed to determine the predisposition of a subject to developing hepatoblastoma is a deneddylation activity assay.

The term "deNEDDylation activity" refers to the ability of:
- a cell lysate comprising a deNEDDylase, preferably NEDP1 or,
- a solution comprising a purified deNEDDylase, preferably NEDP1
to remove a NEDD8 molecule chemically attached to any other molecule.

In a particular embodiment, step I comprises:
a) generating a lysate from a liver biopsy sample from the subject,
b) contacting the liver biopsy lysate, which contains NEDP1, with a NEDP1 substrate comprising a Neural precursor cell Expressed, Developmentally Down-regulated 8 (NEDD8) molecule conjugated to a quenched fluorescent molecule thereby allowing the hydrolysis of the linkage between NEDD8 and the quenched fluorescent molecule due to the deNEDDylation activity of NEDP1 present in the liver biopsy lysate,
c) detecting the fluorescence signal from the unquenched fluorescent molecule as a result of the deNEDDylation.

In a particular embodiment the deNEDDylation assay comprises determining the ability of NEDP1 to remove NEDD8 molecules from a quenched fluorescent molecule.

In a more particular embodiment the deNEDDylation assay comprises determining the ability of NEDP1 to remove NEDD8 molecules from a quenched Rhodamine 110.

As used herein the term "quenched fluorescent molecule" refers to a fluorophore whose fluorescence emission is reduced or supressed as a result of its interaction with a quencher molecule. The state of a fluorescent molecule as quenched is reversible allowing fluorescence to be restored under specific conditions. Such reversibility makes quenched fluorescent molecules useful in the context of cellular assays.

In a particular embodiment the fluorescent molecule is quenched by NEDD8.

In a particular embodiment the quenched fluorescent molecule is Rhodamine 110.

In another particular embodiment the molecule used in the deNEDDylation assay is NEDD8 chemically linked to Rhodamine 110.

In another particular embodiment the molecule used in the deNEDDylation assay is NEDD8 linked to Rhodamine 110 by a chemical bond that can be remove by NEDP1.

In another particular embodiment the molecule used in the deNEDDylation assay is NEDD8 linked to Rhodamine 110 by an isopeptide bond.

The deNEDDylation activity of a specific deNEDDylase, provided as part of a cell lysate or a specific purified recombinant deNEDDylase provided in a solution, is represented as a relative value of the fluorescence emitted by the unquenched fluorescent molecule, as a result of the removal of the quenching molecule NEDD8 by the deNEDDylase, with respect to the background fluorescence.

In a particular embodiment the deNEDDylation activity of NEDP1, provided as part of a cell lysate or as a purified recombinant protein in a solution, is represented as a relative value of the fluorescence emitted by the unquenched fluorescent molecule, resulting from the removal of the quenching molecule NEDD8 by the deNEDDylase, with respect to the background fluorescence.

In a particular embodiment the deNEDDylation activity of NEDP1, provided as part of a cell lysate or as a purified recombinant protein in a solution, is represented as a relative value of the fluorescence emitted by Rhodamine 110, resulting from the removal of the quenching molecule NEDD8 by the NEDP1, with respect to the background fluorescence.

In a particular embodiment the deNEDDylase, preferably NEDP1 is provided as part of a lysate from the tissue originating from a liver biopsy of a subject.

In another particular embodiment the deNEDDylase is provided as a purified protein originating from cell lysate of the biopsy sample of a patient.

In a particular embodiment the cell lysate from the liver biopsy of a subject who could develop hepatoblastoma contains a physiologically relevant concentration of salt and/or a buffer solution.

In an even more particular embodiment the cell lysate from the liver biopsy of a subject who could develop hepatoblastoma contains a physiologically relevant concentration of NaCl and/or TrisHCl.

In a particular embodiment the cell lysate from the liver biopsy of a subject who could develop hepatoblastoma further contains an adequate amount of a reducing agent so that the deNEDDylation activity assay can occur.

In a more particular embodiment the reducing agent is 1,4-Dithiothreitol (DTT).

In an even more particular embodiment the final concentration of DTT is 10mM.

In a particular embodiment the deNEDDylation activity of NEDP1, provided as part of a cell lysate, or as a purified protein as part of a solution is compared to a reference value.

In the context of a method for determining the predisposition of a subject to developing hepatoblastoma, the term "reference value" is understood as the fluorescence value detected in the deNEDDylation assay, when a cell lysate originating from a liver biopsy of a healthy subject, containing NEDP1 is used in the deNEDDylation assay. Alternatively, wherein a purified recombinant NEDP1 is used in the context of a deNEDDylation assay the term "reference value" will be understood as the fluorescence value detected in a, when a wildtype NEDP1 protein is used. As used herein the term "wildtype" refers to a polypeptide sequence or protein that has the amino acid sequence as it would result from the translation of an mRNA molecule originating from a gene under normal conditions. The wildtype protein displays the expected structure and carries out its expected biological activity under normal physiological conditions.

In a particular embodiment the reference value of the deNEDDylation assay corresponds to the values obtained when a plurality of cell lysate from a liver biopsy of healthy patients are analysed.

In a particular embodiment the fluorescence values of the deNEDDylation assays using the cell lysate of a liver biopsy of a patient who could develop hepatoblastoma are normalized to the reference value.

The second step of the method of the fourth aspect comprises comparing the deNEDDylation activity in the liver biopsy sample with a reference value.

In a particular embodiment the normalized values from the deNEDDylation activity assays using the cell lysate of a liver biopsy of a patient who could develop hepatoblastoma are compared to the reference value.

The analysis of the comparison, the terms "increased and "decreased", as well as the statistical tests performed to determine whether there is a statistically significant difference have been previously described and applied to this aspect of the invention.

In a particular embodiment when the deNEDDylation activity of the cell lysate of a liver biopsy of a subject who could develop hepatoblastoma or hepatocellular carcinoma compared to the reference value is deemed significantly decreased, it is indicative that the subject has a predisposition to developing hepatoblastoma or hepatocellular carcinoma.

In a particular embodiment the method to determine the predisposition of a subject to develop hepatoblastoma may also be employed for determining the predisposition of a subject to developing hepatocellular carcinoma. All the previously described aspects relating to the method to determine the predisposition of a subject to developing hepatoblastoma also apply to the method to determine the predisposition of a subject to develop hepatocellular carcinoma.

### EXAMPLES

### MATERIAL AND METHODS

### Human samples and clinical information

All human samples from pediatric patients with HB and main clinical features that have been used in the present project were obtained after informed consent of the patients and in accordance with the Ethical Code of the World Medical Association, Helsinki Declaration and upon approval of the hospital Ethics Committee. Fresh-frozen (FF) and formalin-fixed paraffin embedded (FFPE) tumor and adjacent non-tumor tissue samples were analyzed from the 16 patients with HB; the main clinical and pathological characteristics are summarized in Table 1.

### Transcriptomic Data of human samples

RNA-sequencing and Human Transcriptome Array (HTA, Thermo Fisher Scientific) databases from of 32 paired HBs and surrounding non-tumor tissues previously published by Carrillo-Reixach et al, 2020 (Carrillo-Reixach J, et al. J Hepatol. 2020 Aug;73(2):328-41) were studied.

### Statistical analysis

Statistical analysis was performed with SPSS v.26 (Chicago, IL, USA) and GraphPad Prism v.10.1.0 (La Jolla, CA, USA) software. Appropriate two-side statistical tests were applied when necessary: qualitative variables (Chi-square and Fisher Exact tests) and quantitative (T-student). The Shapiro-Wilk test was performed for the study of the normality of the data. For event free survival (EFS) analysis, Kaplan-Meier's method and Log rank test were performed to compare differences between curves.

### Preclinical Studies and Animal Maintenance

The animal procedures were performed in accordance with the European Research Council for animal care and use, and the National Institute of Health guide for care and use of Laboratory animals. The maximal authority of the Country Council of Bizkaia and the Institutional Animal Care and Use Committee of CIC bioGUNE have approved all the animal procedures. Male adult (three-month-old) C57BL/6J mice were acquired from Charles River Laboratories and accommodated into the AALAC-accredited CIC bioGUNE animal facilities and maintained at 21±1°C, 45±10% humidity and 12/12h light/dark cycle. The animal maintenance was based on ad libitum access to water and the respective diet.

### ^{Bio}NEDD8 transgenic mice model

^{Bio}NEDD8 transgenic mice were used to identify proteins undergoing post-translational modification and to assess their modulation following NEDP1 overexpression. These mice possess a random genetic insertion of NEDD8 sequences conjugated with a biotin tag, allowing for targeted analysis of modified proteins (Litten JB, Tomlinson GE. Oncologist. 2008 Jul 1;13(7):812-20).

### Induction of preclinical model of HB

Hydrodynamic vein tail injection was performed in 6-week-old mice the combination of plasmids which contains the mutated sequence of CTNNB1N90 (β-catenin) and YAP-1S127A (Yes1 Associated Transcriptional Regulator) (10 ng/µl) together with SB (Sleeping Beauty) (0,8 ng/µl) transposon as it was described in Tao et al., 2014 (Schnater JM, et al. Cancer. 2003 Aug 15;98(4):668-78). With the HD method, plasmids are mainly delivered into the liver of the mice where the transposon recognizes the sequences flanking these two genes, and they are incorporated into the liver genome of the mice. After echography analysis every 3 weeks, tumors reached a considerable size at week number 9 and mice were randomly divided in two groups of treatment. One of the groups were treated with 1·10^10 ucf from Adenovirus which overexpress Gfp as a control group, and the other group with 1·10^10 ucf from adenovirus which contain the overexpression of the *Senp8* mouse sequence (to overexpress NEDP1 protein) by vein tail injection once a week the following 4 weeks provided by VectorBuilder. At 13 weeks after the hydrodynamics mice were sacrificed. At the time of sacrifice, liver samples were rapidly split into several pieces, frozen in liquid nitrogen and stored at -80°C for subsequent protein and RNA analysis. Half part from the left lobe was formalin-fixed for histology and immunohistochemistry. In addition, sub-mandibular blood was collected once every week in order to follow up on the development of the disease. Finally, blood samples were obtained at the time of sacrifice to determine biochemical parameters such as alanine aminotransferase (ALT) and aspartate aminotransferase (AST). All the work was performed under the legal regulation of security manage of this biological agent.

### Cell Lines

The Human hepatoma cell line HepG2 was purchased from the American Type culture collection (ATCC) and HepT1 was kindly provided by Dr. Matias Avila (Center for Applied Medical Research CIMA, University of Navarra, Spain). In addition, the immortalized primary normal liver cells, THLE-2, were purchased from ATCC (ATCC^{®} CRL-2706TM). Finally, a primary HB-284 cell line founded from patients' derived xenograft was kindly provided by Dr. Matias Avila (Center for Applied Medical Research CIMA, University of Navarra, Spain) and previously described in D. Nicolle et al., 2016 and Eloranta et al., 2020 (Linabery AM, Ross JA. (1992-2004). Cancer. 2008 Jan 15;112(2):416-32; Kremer N, et al. Curr Opin Pediatr. 2014 Jun;26(3):362-9).

### deNEDDylating Activity Assay (in vitro and in vivo)

Briefly, deNEDDylating activity assay was performed in THLE-2 cell line, HB cell lines without and 48h post transfection, and also in liver tissue. For tissue samples, 25mg of liver tissue is homogenized in tissue lysis buffer (0.5% NP40, 150mM NaCl, 50mM Tris HCl (pH 7.4), 0.1% SDS) using mortar and pestle and subsequently sonicated, in two rounds (10 sec on - 10 sec off) on ice. The total extracted lysate was centrifuged (13000 rpm, 20 minutes, 4°C). The supernatant is ultracentrifuged (50000rpm, 30 minutes. 4°C). Cells were lysed in a cell lysis buffer (150mM NaCl, 20 mM Tris HCl (pH 7.4)) by sonication at 20%power in 3 cycles by 10 seconds. Then the supernatant was centrifuged (13000 rpm, 15 minutes, 4°C). In both cases, the final supernatant was quantified for total protein concentration using Micro BCA Protein Assay Kit (Thermo Scientific). Afterward, 30 µg of protein per sample was incubated with NEDD8 Rhodamine 110 (South Bay Bio) fluorescent substrate in 30uL of buffer reaction mix (0.5uM NEDD8 Rhodamine 110, 10mM DTT and lysis buffer). Kinetic measurements every 2min for 45min in total at 37°C were performed by using a luminescent spectrophotometer -λ excitation of 485 nm and λ emission of 535 nm (the experimental design was adapted from a protocol kindly provided by Dr. Dimitris Xirodimas from Montpellier University).

### Cells transfection

### Plasmid cDNA transfection

Lipofectamine 2000 (Invitrogen) was used as a reagent for cell lines (HepT1, HepG2 and THLE-2) transient transfection according to manufacturer's instructions.

DNA-Lipofectamine complex formation: Lipofectamine 2000 (1.25 µl/1 µg DNA) was diluted in 500 µl of OPTI-MEM (Gibco) medium for transfections in 6 well plates and in 100 µl for transfections in 24 well culture plates and incubated for 5 min. In particular, HepT1 cells were sensitive to Lipofectamine, hence 40% of reduced concentrations were used (results based on IC50 calculation). After incubation, the mixture was added to 500 µL of OPTI-MEM (100 µL for 24 well plates) containing plasmid DNA (1 to 6 µg depending on the experiment) and incubated for at least 20 min at room temperature (RT) to allow for the formation of the DNA-Lipofectamine complexes.

Cells transfection: DNA-lipofectamine complexes previously formed were added to the culture plates containing the corresponding culture medium with 5% FBS but without antibiotics and with the cells in suspension. The mixture with cells was left overnight and the culture media was replaced the next morning with a fresh culture medium supplemented with antibiotics. The cells were collected 48h later transfection, to be either lysed or subjected to the corresponding treatment depending on the experiment. Transfection efficiency was confirmed by Western blotting or RNA expression analysis.

In the PDX cell line (HB-284), the reagent was replaced by JetPrime (Polyplus transfection) due to the sensitivity to Lipofectamine 2000 (Invitrogen) and used according to manufacturer's instructions. Plasmid DNA (0.5 to 4 µg depending on the experiment) was diluted in 200uL JetPrime Buffer (Polyplus transfection) for transfections in 6 well plates and in 50 uL for transfections in 24 well culture plates and mixed by vortexing. Then, JetPrime reagent (Polyplus transfection) was added (2 uL/1 ug DNA), mixed by a brief vortexing and incubated at room temperature (RT) for 10 minutes. The transfection mix were added to the culture plates containing the corresponding complete culture medium and with the cells in suspension. The mixture with cells was left overnight and the culture media was replaced the next morning with a fresh culture medium.

### Plasmid construction

Most plasmids employed in the present project were kindly provided by Dr. Dimitris Xirodimas from Montpellier University, including the plasmid constructions: pcDNA3.0-NEDP1-V5 encoding full-length human NEDP1, pcDNA3.0 empty vector as transfection control, pcDNA3.0-NB2-FLAG encoding the nanobody isoform 2, pcDNA3.0-NB9-FLAG encoding the nanobody isoform 9 and pcDNA3.0-NB10-FLAG encoding the nanobody isoform 10 which are an inhibitor of NEDP1 activity (6). The pcDNA3-Myc3-CAND1 plasmid was a gift from Yue Xiong (Addgene plasmid #19948; http://n2t.net/addgene:19948; RRID: Addgene_19948) (Abidi N, Xirodimas DP. Endocr Relat Cancer. 2015 Feb;22(1):T55-70).

### RNA isolation and retro-transcription

Total RNA from liver tissue or cell culture was isolated using Trizol reagent (Invitrogen)/ Chloroform (SigmaAldrich). In the case of cell mRNA extraction, 5 µL of Glycogen (Invitrogen) were used in order to facilitate RNA precipitation. After analyzing the integrity and the concentration using Nanodrop ND-100 spectrophotometer, 1-2 µM of total RNA was treated with DNAse I (Invitrogen) and cDNA was synthesized using M-MLV Reverse Transcriptase (Invitrogen) in the presence of random primers, RNAase OUT and ribonucleotides. Resulting cDNA was diluted 1/10 in the case of cell lines and 1/20 in the case of tissue.

### Real time quantitative PCR (RT-qPCR)

Quantitative real-time (RT-PCR) was performed using SYBR^{®} Select Master Mix (Applied Biosystems) and the QuantStudio^{™} 5 Real-Time PCR (Applied Biosystems). 2 µL of cDNA was used and including the specific primers for a total reaction volume of 7 µL in a 384-well plate (Applied Biosystems). All reactions were performed in triplicate. PCR conditions for the primers were optimized and 40 cycles with a melting temperature of 60 °C and 30s per step were used. *Homo sapiens* and *Mus musculus* primers were designed using Primer-Blast (https://www.ncbi.nlm.nih.gov/tools/primer-blast/) and synthesized by Sigma Aldrich. The calculations were based on the differential of ΔCt cycles (expression) between the housekeeping (GAPDH, ARP, HPRT1) and the target gene. Primers sequences are described for humans and for mice (Supplemental Table II and Supplemental Table III).

### Protein extraction and analysis

Approximately, 50 mg of liver tissue was homogenized by using Precellys-24 (Bertin Technology). The lysis buffer (NaH2PO4 1.6 mM, Na2HPO4 8.4 mM, 0.1% Triton X-100, NaCl 0.1 M, 0.1% SDS, 0.5% sodium azide) used to homogenize liver tissue was supplemented with protease and phosphatase inhibitor cocktail (Roche), 2-lodocetamid 20 mM (Merck) and N-Ethylmaleimide 20 mM (Sigma-Aldrich). The lysate of the total extracted protein was centrifuged (14000 rpm, 30 minutes, 4°C) and the supernatant was quantified for total protein content by the Bradford protein assay (BioRad) or Micro BCA Protein Assay Kit (Thermo Scientific). Protein extracts from each sample were boiled at 95°C for 5 minutes in SDS-PAGE sample buffer (50 mM Tris-HCl pH 6.8, 500 mM β-mercaptoethanol, 50% glycerol, 10% SDS, bromophenol blue). 7-20 µg of protein from each sample were separated by sodium dodecyl sulphate-polyacrylamide gel electrophoresis (SDS-PAGE) in 8-15% acrylamide gels, using Mini-PROTEAN Electrophoresis System (Bio Rad). Protein gels were transferred onto nitrocellulose blotting membranes (GE Healthcare Life Science). Membranes were blocked with 5% non-fat milk in TBS PH 8 containing 0.1 % Tween-20 (Sigma Aldrich) (TBST-0.1%) for 60 min at RT, washed 3 times with TBST and incubate overnight at 4°C with commercial antibody (1:1000), and incubated with secondary antibody conjugated with horseradish peroxidase (HRP) 1 -2h RT (1:5000, anti-rabbit-IgG-HRP-linked and anti-mouse IgG-HRP linked, Cell Signalling Technology). Immunoreactive proteins were detected by using Clarity Western ECL Substrate reagent (BioRad) and exposed in iBright CL750 system (Invitrogen). Antibodies and conditions are described in Supplemental Table IV. Densitometry analysis was performed using Image J software (NIH).

### Proteomic analysis

### Tryptic digestion

Gel bands were washed in milli-Q water. Reduction and alkylation were performed using dithiothreitol (10 mM DTT in 50 mM ammonium bicarbonate) at 56 °C for 20 min, followed by iodoacetamide (50 mM iodoacetamide in 50 mM ammonium bicarbonate) for another 20 min in the dark. Gel pieces were dried and incubated with trypsin (12.5 µg/ml in 50 mM ammonium bicarbonate) for 20 min on ice. After rehydration, the trypsin supernatant was discarded. Gel pieces were hydrated with 50 mM ammonium bicarbonate and incubated overnight at 37°C. After digestion, acidic peptides were cleaned with TFA 0.1% and dried out in a RVC2 25 speedvac concentrator (Christ). Peptides were resuspended in 10 µl 0.1% FA and sonicated for 5 min prior to analysis.

### Mass spectrometry analysis

Samples were analyzed in a timsTOF Pro with PASEF (Bruker Daltonics) coupled online to an Evosep ONE liquid chromatograph (Evosep). 200ng were directly loaded onto the Evosep ONE and resolved using the 60 samples-per-day protocol.

DIA data was processed with DIA-NN software for protein identification and quantification using default parameters. Searches were carried out against a database consisting of *Mus musculus* protein entries from Uniprot in library-free mode. Carbamidomethylation of cysteines was considered as fixed modification and oxidation of methionines as variable modification. Data was loaded onto Perseus platform for data processing (log2 transformation, imputation) and statistical analysis (Student's t-test). Proteins with a p<0.05 were considered for further analyses and discussion.

### Tissue staining assay

### Hematoxylin and eosin (H&E)

Paraffin slices were stained with Harris Hematoxylin (Bio-Optica) for 15 min, washed in running tap water for 5 minutes, differentiated in 0.5% HCl for 1 s and stained with aqueous Eosin solution (Sigma-Aldrich) for 15 minutes. The samples were washed in running tap water for 5 minutes, dehydrated in graded alcohol solutions to 100% and mounted in DPX mounting medium.

### Immunohistochemistry

The procedures followed to develop the immunohistochemistry study were according to standard protocols described in EnVision+System HRP (Dako). Formalin-fixed paraffin embedded liver biopsies were sliced in 5-µm thick sections with HistoCore MULTICUT microtome (Leica), dewaxed with Histo-Clear I solution (Electron Microscopy Sciences), hydrated and unmasked using antigen retrieval solutions with a Dako cytomation.

The samples were incubated with the primary antibodies for NEDD8 and NAE1 overnight at 4°C followed by the corresponding ImmPRESS^{®} secondary polymer (Vector Laboratories, MP-7451 and MP-7452) for 30 minutes. The detection was chromogen, using Vector Vip purple substrate (Vector Laboratories). All the samples were counterstained with Mayer's Hematoxylin (Sigma-Aldrich), dehydrated, cleared in Histo-Clear I solution and mounted with DPX mounting medium (Sigma Aldrich). The antibodies and the conditions that have been used can be found in Supplemental Table V.

### Data analysis

To analyze all histological and immunofluorescence samples four to ten random images per sample were taken with 10x, 20x or 40x objectives using either Leica DM750 optical microscopy with a digital colour camera (Leica ICC50W) or the Axioimager D1 microscope (Carl Zeiss AG, Germany). FIJI (Image J) software was used to quantify the total stained area for each image.

### Biochemical analysis

Serum transaminases were analyzed with Selectra Junior Spinlab 100 analyzer (Vital Scientific, Dieren, Netherland) according to manufacturers' suggested protocol.

### Respiration analysis

Respiration analyzes were performed in cells using a Seahorse Bioscience XF24-3 Extracellular Flux Analyzer. Oxidative phosphorylation was measured as the oxygen consumption rate (OCR), which is the rate change of dissolved O2. Anaerobic glycolysis was measured as the extracellular acidification rate (ECAR), which is the rate change of pH in medium immediately surrounding adherent cells. All measurements were performed at 37°C.

The cells were seeded in XF24 cell culture plate (Seahorse Bioscience) at 1.5 x 10⁴ cells per well in a final volume of 100 µL of cell transfection mix with EV and NEDP1, respectively. The mixture with cells was left overnight and the culture media was replaced the next morning with 500uL of fresh culture medium supplemented with antibiotics. The day before the assay, the XF24 sensor cartridges (102070-001, Agilent) were hydrated with Seahorse XF calibrant solution (102070-001, Agilent) overnight at 37°C in a 0% CO2 incubator. The assay was performed 48h after transfection, growth medium was removed and replaced with 500 µl of assay medium prewarmed to 37°C, composed of DMEM without bicarbonate containing 1mM glucose, 1 mM sodium pyruvate, 2 mM L-glutamine, and cultured at 37°C in room air. Measurements of OCR and ECAR were performed after equilibration in assay medium for 1h. After OCR and ECAR baseline measurement, sequential injections were performed through ports in the XF Assay cartridges. The following pharmacologic inhibitors were used at oxidative phosphorylation assay: oligomycin (6 µM), an inhibitor of ATP synthase, which allows the measurement of ATP coupled oxygen consumption through OXPHOS; carbonyl cyanide 4-trifluoromethoxy-phenylhydrazone (FCCP) (3 µM), an uncoupling agent that allows maximum electron transport, and therefore a measurement of the maximal OXPHOS respiration capacity; and a combination of rotenone (0.5 µM)/ antimycin (0.5 µM), a mitochondrial complex I inhibitor. To anaerobic glycolysis a combination of rotenone (0.5 µM)/ antimycin (0.5 µM) to inhibit the mitochondrial electron transport chain, and 2-DG (2-deoxy-D-glucose) (0.18mg/mL) a glucose analogue to inhibit glycolysis were added. The OCR and ECAR was normalized by the number of cells, which were estimated by crystal violet.

### Caspase-3-activity assay

Caspase-3 activity assay was performed in HB cell lines after 48h or 72h of the transfection. Cells were lysed in a Lysis buffer without protein inhibitors (0.5 M HEPES, pH 7.4, CHAPS hydrate 10% 3, 0.5 M EDTA, 2.5M dithiothreitol) and protein quantified by Bradford protein Assay (BioRad). Afterward, 20 µg of protein per sample was incubated with AC-DEVD-AMC fluorescent substrate in buffer reaction mix (ENZO Life Sciences,) at 37°C. Kinetic measurements at 0 h, 1h, 3h, and 4h were performed by using a luminescent spectrophotometer -λ excitation of 390 nm and λ emission of 510 nm. Cells treated for 24h with 5 µg/mL (to HepT1 and HepG2) or 8.5 µg/mL (to HB-284) of cisplatin in NaCl 0.9% (a well-established apoptosis inductor, Selleckchem) were used as a positive control.

### Crystal violet staining

Cell viability and proliferation studies were done using crystal violet staining based on the staining of alive cells which are attached to the culture plate instead of dead cells which are detached. For proliferation studies, 5 × 10⁴ HB transfected with EV and NEDP1 cells were seeded in 24-well plates and the time points were collected after 24, 48 and 72 hours. Cells were washed with PBS twice and fixed in ice-cold 4% paraformaldehyde solution in PBS (Santa Cruz Biotechnology) for 10 min at room temperature. Cells were washed with PBS twice and incubated with a 0.1% crystal violet (Sigma-Aldrich) solution in 20% methanol for 40 min at room temperature. The staining was discarded, and the plates were rinsed using distilled water and left to dry overnight. Crystals were resuspended in 10% acetic acid for 1 hour at room temperature. The resulting colour was measured using absorbance at 595nm in the Victor Nivo spectrophotometer (PerkinElmer).

### Mitochondrial ROS

Mitochondrial ROS was measured using MitoSOX^{™} Red reagent (Life Technologies), following manufacturer's instructions. Briefly, a total of 2,5×10⁴ HB transfected with EV and NEDP1 cells were seeded over 12-mm coverslips in 24-well plates for 48h. After finishing the experiment cells were incubated with MitoSOX reagent (2.5 µM, 10', 37°C in a CO2 incubator) in normal culture medium. Then, cells were washed with PBS and fixed in ice-cold 4% paraformaldehyde solution in PBS (Santa Cruz Biotechnology) for 10 min at room temperature. Covers were mounted with Fluoroshield Mounting Medium with DAPI (Dako, USA). Pictures were taken with an Axio Imager D1 Upright Fluorescence Microscope (Carl Zeiss AG, Jena, Germany). Quantification of fluorescence intensity was performed using FIJI (Fiji is just image J) https://imagej.net/Fiji. A total of 5 pictures from each cover were taken. The nucleus number from each image was used to normalize intensity.

### Mitochondrial mass

Mitochondrial mass was measured using MitoTracker Green FM (Life Technologies), following manufacturer's instructions. Briefly, a total of 2,5×10⁴ HB transfected with EV and NEDP1 cells were seeded over 12-mm coverslips in 24-well plates for 48h. After finishing the experiment cells were incubated with MitoTracker reagent (100nM, 30', 37°C in a CO2 incubator) in normal culture medium. Then, cells were washed with PBS and fixed in ice-cold 4% paraformaldehyde solution in PBS (Santa Cruz Biotechnology) for 10 min at room temperature. Covers were mounted with Fluoroshield Mounting Medium with DAPI (Dako, USA). Pictures were taken with an Axio Imager D1 Upright Fluorescence Microscope (Carl Zeiss AG, Jena, Germany). Quantification of fluorescence intensity was performed using FIJI (Fiji is just image J) https://imagej.net/Fiji. A total of 5 pictures from each cover were taken. The nucleus number from each image was used to normalize intensity.

### Histopathological analysis

An independent pathologist evaluated hematoxylin and eosin-stained tissue sections by scoring them according to the quantity of tumor clusters visible within each 9mm2 field of view, the percentual quantity of tumoral infiltration. The scores for each category are stablished as followed: a score of 1 indicates infiltration fewer than 15%, a score of 2 indicates infiltration between 16-30% a score of 3 indicates infiltration between 31-60% and a score of 4 indicates infiltration higher than 61%.

### Streptavidin beads pull down assay (BioNEDD8)

The procedure for isolating biotinylated proteins from ^{Bio}NEDD8 transgenic mice was conducted as described in (8). Approximately 250 mg of liver tissue expressing the (bio-NEDD8)-BirA construct (or just BirA) was homogenized in 1.5 mL of lysis buffer under denaturing conditions (including a protease inhibitor cocktail from Roche and N-Ethylmaleimide from Sigma-Aldrich) by using Precellys-24 (Bertin Technology). A small volume of this total protein fraction (INPUT) was set aside. The liver extracts were then incubated with 250 µL of NeutrAvidin-Agarose beads (ThermoScientific), previously equilibrated with binding buffer, for 2 hours at room temperature in movement. The samples were centrifuged at 2000 rpm for 5 minutes, and the supernatant was collected (a small volume of the flow-through fraction was kept.) The beads were transferred to 1.5 mL Eppendorf tubes and subjected to a series of washes: twice with 1 mL of WB1, three times with WB2, once with WB3, three times with WB4, once with WB1, once with WB5, three times with WB6, and finally and extra wash with WB7 was performed. To ensure thorough cleaning and removal of residual liquid between washes, the tubes were centrifuged at 2000 rpm for 1 minute after each wash. Finally, the beads were mixed with 125 µL of elution buffer (boiling buffer containing 500 mM β-mercaptoethanol and 100 mM DTT) for 10 minutes at room temperature (RT) in a LabRoller rotator, followed by boiling at 95°C for 5 minutes. The eluted sample was separated from beads using specialized microcentrifuge filter (VivaClean Mini 0.8 µL m PES).

Buffer compositions are listed as follows: Lysis buffer contained 8 M urea, 1% SDS, and 50 mM N-ethylmaleimide in PBS, including a protease inhibitor mixture (Roche Applied Science); binding buffer contained 3.15 M urea, 1 M NaCl, 0.25% SDS, and 50 mM N-ethylmaleimide in PBS; WB1 contained 8.4 M urea and 0.25% SDS in PBS; WB2 contained 6 M guanidine HCl in PBS; WB3 contained 6.3 M urea, 1 M NaCl, and 0.2% SDS in PBS; WB4 contained 4.2 M urea, 1 M NaCl, 10% isopropanol, 10% ethanol, and 0.2% SDS in PBS; WB5 contained 8 M urea and 1% SDS in PBS; WB6 contained 2% SDS in PBS; WB7 is composed by 8M urea in PBS, and elution buffer contained 4× Laemmli buffer and 100mM DTT.

### Protein immunoprecipitation (IP)

NEDD8 was isolated from total protein extracts through immunoprecipitation using an anti-NEDD8 antibody (ab81264, Abcam) that was covalently attached to Protein G Sepharose resin (Cytiva, Cat#GE17-0618-01) to avoid the elution of the antibody along with the target protein, following the method outlined at (9). Protein A/G PLUS-Agarose (2003, Santa Cruz Biotechnology) was first washed five times with 10 column volumes of PBS containing 0.1% sodium azide, followed by centrifugation (5,000 rpm, 5 min, 4°C) to remove the storage solution. For each reaction, 100 µl of the Protein A/G PLUS-Agarose slurry was incubated with 2 µg of NEDD8 antibody or Purified Mouse IgG1 κ Isotype Control antibody (557273, BD Pharmingen) in 1 ml of 0.1% sodium azide-PBS overnight at 4°C with rotation to allow antibody binding to the resin. The beads were centrifuged (2,500 rpm, 5 min, 4°C), washed twice with 1 ml of sodium borate buffer (200 mM boric acid, 3 M NaCl, pH 9.0), and incubated with 1 ml of 50 mM dimethyl pimelimidate dihydrochloride (DMP) (80490, Sigma-Aldrich) in sodium borate buffer for 30 minutes at room temperature with rotation. After another centrifugation (2,500 rpm, 5 min, 4°C), the beads were washed twice with sodium borate buffer and twice with 1 ml of 200 mM ethanolamine, pH 8.0, then incubated for 2 hours in the dark at room temperature to neutralize unreacted DMP. The beads were centrifuged (2,500 rpm, 5 min, 4°C) and washed twice with 1 ml PBS, then with 1 ml of 200 mM glycine, pH 2.5, followed by two more washes with PBS to eliminate non-crosslinked antibody. Simultaneously, cells were lysed in 50 mM Tris, pH 8.5, 150 mM NaCl, 5 mM EDTA, 1% Nonidet P-40 (NP-40), and 1 mM phenylmethylsulfonyl fluoride (PMSF), and protein extraction was conducted as previously described. Protein concentration was measured using the Micro BCA Protein Assay Kit (23235, Thermo Fisher Scientific). Next, 600 µg of protein were mixed with antibody-bound resin in 500 µl of lysis buffer and incubated for 2 hours at 4°C with agitation. The beads were then centrifuged (5,000 rpm, 5 min, 4°C), washed three times with lysis buffer, and mixed with 35 µl of 5x Laemmli loading buffer. After rotating for 5 minutes at room temperature and boiling the samples at 95°C for 5 minutes, they were centrifuged again (13,000 rpm, 12 min, room temperature), and the supernatant containing the immunoprecipitated proteins was analysed by Western blot.

### Statistical Analysis

Prism 8 (GraphPad Software, version 8.4.0) was used to perform statistical analysis. One-way analysis of variance (ANOVA) followed by Tukey (comparing all pairs of columns) was used in case of three or more groups, while Student's t-test was used in case 2 groups. Grubbs' test was performed to determine the significant outliers. A p<0.05 was considered statistically significance. Statistical parameters are reported in the figure legends.

### RESULTS

### Disturbances of the NEDDylation Pathway in Hepatoblastoma

To underscore the critical role of NEDDylation in tumor development, transcriptomic data from 32 human HB samples were analyzed compared to healthy liver tissue ( Carrillo-Reixach J, et al. J Hepatol. 2020 Aug;73(2):328-41). As shown in Figure 1, there was an upregulation in key components of the NEDDylation machinery, including catalytic subunit UBA3 from E1 activating enzymes (NAE1), E3 ligases (CBL, DCUN1D1, DCUN1D3), and deconjugating enzymes that have been reported to possess dual activity for Ubiquitin and NEDD8 (UCHL1, UCHL3, USP21). The increased expression of NAE1, an essential enzyme that activates NEDD8, mirrors patterns observed in other liver cancers such as hepatocellular carcinoma (HCC) and cholangiocarcinoma (CCA)(Yu J, et al.. Cancer Med. 2018 Jul 30;7(7):3363-72, Olaizola P, et al. J Hepatol. 2022 Jul;77(1):177-90). These findings suggest an important transcriptional regulation of genes related with NEDDylation pathway in HB patients.
Protein analysis in the liver sections of human HB samples revealed a notable increase in NEDD8 and NAE1 levels within tumor regions compared to adjacent non-tumoral tissues (Figure 2 A, Table 1).

**Table 1. Hepatoblastoma Patient Cohort Summary. Characterization of the cohorts of Hepatoblastoma patients used for the tumor immunohistochemically, Western Blot and deNEDDylating activity. Data are represented as the average and standard error of the mean (SEM).**

| | ***Patients included (n=16)*** |
|---|---|
| **Age, months (median, [range])** | 30 [1-158] |
| **Gender (M/F)** | 11/5 |
| **Serum AFP, ng/mL (range)** | 5,332 - 1,500,000 |

| **Clinical classification:** | |
|---|---|
| **CHIC-HS (VL-L/I/H)** | 8/2/6 |

| **Tumor characteristics:** | |
|---|---|
| **PRETEXT stage (I/II/III/IV)** | 2/5/7/2 |
| **Vascular Invasion (Y/N/NA, %)** | 1/11/4 (8%) |
| **Multifocality (Y/N, %)** | 2/14 (12%) |
| **Metastasis at diagnosis (Y/N, %)** | 3/13 (19%) |

| **HB histology:** | |
|---|---|
| **Epithelial/Mixed** | 10/6 |
| **MEC: Fetal/Non-Fetal^{a}** | 5/11 |
| **Follow-up, months (mean, [range])** | 56 [12-138] |
| **Outcome: cancer-related deaths or tumor recurrence** | 2 (12%) |
| ^{a}Non-fetal includes crowded fetal, macrotrabecular and embryonal histological subtypes. Abbreviations: CHIC-HS, Children's Hepatic tumors International Collaboration-Hepatoblastoma Stratification (VL-L, very low; or low; I, intermediate and H, high risk); F, female; M, male; MEC, Main Epithelial Component; NA, non-available; N, No; PRETEXT, PRETreatment EXTent of disease; Y, yes. | |

These findings were further corroborated by Western blot assays (Figure 2B), which consistently demonstrated elevated protein levels in tumor. Interestingly, despite the absence of significant transcriptional changes, NEDP1 protein levels were reduced in HB samples in comparison to the values of NEDP1 in a healthy liver (The Human Protein Atlas) (Figure 2B). Importantly, NEDP1 activity assessed in the liver of HB patients was significantly diminished, closely mirroring the reduced protein levels (Figure 3 A and B).

Preclinical HB models, crucial for studying tumor biology and developing novel therapies, have been refined to more accurately replicate the disease progression observed in clinical settings. In the CTNNB1N90 YAP-1S127A HB mouse model, significant alterations in the NEDDylation pathway were detected (Figure 4, Figure 5) ( Tao J, et al. Gastroenterology. 2014 Sep;147(3):690-701). Specifically, tumors exhibited an increase in global NEDDylation across the proteome (Figure 6A). This disturbance was accompanied by elevated NAE1 levels and a marked reduction in NEDP1, consistent with findings from human HB (Figure 6 A and B). Additionally, deNEDDylating activity is reduced in the livers of this *in vivo* HB model, similar to what is observed in patient samples (Figure 5 and Figure 6B).

These results collectively highlight hyperactivation of the NEDDylation pathway and reduction in NEDP1 levels and activity in both HB patients and a HB mouse model.

### NEDP1 Activity Regulates Proliferation in Hepatoblastoma Cells

To further elucidate the effects of NEDDylation in HB cells, global NEDDylation levels were assessed in HepT1 and HepG2 cell lines, with non-transformed human hepatocytes (THLE-2) as a baseline for comparison.

Consistent with previous observations in HB patients and mouse models, an increase in global NEDDylation was noted in the HB cell lines compared to THLE-2 cells (Figure 7A). This upregulation was accompanied by decreased levels of NEDP1 and a marked reduction in deNEDDylating activity (Figure 7 A, B, C), highlighting the association of NEDP1 depletion with accumulation of NEDD8 conjugates in the pathogenesis of HB tumors. Furthermore, transcriptomic analyses identified significant changes in the expression of pivotal enzymes involved in the NEDDylation pathway within the tumor cells (Figure 8).

To explore the potential role of NEDP1 activity in cancer progression, nanobody (NB) constructs previously developed to specifically inhibit NEDP1 activity in non-tumorigenic THLE-2 hepatocytes were employed. These nanobodies, which are small proteins derived from the unique antigen-binding domains of camelid antibodies, exhibit precise target specificity and inhibitory potency (Abidi N, et al. bioRxiv [Internet]. 2020 Jan 1;2020.03.20.999326).

From the collection of developed NBs, those exhibiting the highest level of inhibitory activity against NEDP1 were selected for further study (Figure 9 A and C). The inhibition of NEDP1 by these nanobodies resulted in an accumulation of NEDD8 conjugates and the activation of cell proliferation signaling pathways, notably Phospho-ERK1/ERK2 at Thr202 and Tyr204 (Figure 9 B). This was associated with a marked increase in cell proliferation when NEDP1 activity was suppressed (Figure 10).

In contrast, re-establishing deNEDDylase activity in HB cells through the overexpression of NEDP1 (Figure 11, Figure 12) triggered an apoptotic response, as determined by enhanced caspase-3 activity and the accumulation of cleaved PARP protein (Figure 13).

These outcomes highlight the role of NEDP1 as a putative tumor suppressor, modulating NEDDylation dynamics within the cell.

### Metabolic Disruption in Tumor Cells Following NEDP1 Overexpression

Cancer cells are characterized by accelerated proliferation rates and augmented metabolic activity to support their increased energy requirements (Pavlova NN, Zhu J, Thompson CB. Cell Metab. 2022 Mar;34(3):355-77). Notably, pharmacological inhibition of NEDDylation, which simulates the effect of elevated NEDP1 expression by diminishing NEDD8 conjugates, has been observed to provoke metabolic shifts in both diseased and healthy states (Barbier-Torres L, et al. Oncotarget. 2015 Feb 10;6(4):2509-23). Considering this, it was investigated how restoring NEDP1 activity affects the metabolic processes of HB tumor cells, particularly glycolysis and oxidative phosphorylation (OXPHOS). After NEDP1 overexpression, a notable reduction in both extracellular acidification rate (ECAR) and oxygen consumption rate (OCR) across the cell lines (Figure 14 and Figure 15) was observed. These changes could be linked to a significant decrease in mitochondrial integrity, as evidenced by diminished MitoTracker green fluorescence, and a marked escalation in reactive oxygen species (ROS), as shown by increased MitoSOX fluorescence (Figure 16). Therefore, the mitochondrial dysfunction detected in HB cells could be intricately connected to the apoptosis activation previously noted with NEDP1 overexpression.

### NEDP1 Functions as a Tumor Suppressor in Patient-Derived Xenograft Cells

Cell lines derived from PDX models have been established to deepen our understanding of HB considering limited human sample availability and to minimize animal use (Abdolahi S, et al., J Transl Med. 2022 May 10;20(1):206, Zanella ER, et al. Nat Rev Clin Oncol. 2022 Nov 23;19(11):719-32). Specifically, metastatic cell line from an 83-month-old child, characterized by its embryonal cellular composition (HB-284), was selected to investigate the tumor-suppressive role of NEDP1 ( Eloranta K, et al. Front Oncol. 2020 Jul 17;10). Compared to THLE-2 hepatocytes, HB-284 cells displayed an abundance of NEDD8 conjugates and elevated NAE1 protein levels, alongside diminished NEDP1 expression (Figure 17). These findings align with the observed decrease in deNEDDylating activity compared to non-tumoral cells (Figure 18).

The overexpression of NEDP1 into HB-284 cells led to reduced global NEDD8 conjugate levels (Figure 19). The presence of NEDP1 was also associated with a significant decrease in cell proliferation rates (Figure 20 A). Furthermore, the restoration of NEDP1 activity in HB-284 cells resulted in enhanced apoptosis, evidenced by increased caspase-3 activity and PARP cleavage (Figure 20 B and C).

Moreover, HB-284 cells with upregulated NEDP1 demonstrated compromised metabolic processes, including decreased extracellular acidification rate (ECAR) and oxygen consumption rate (OCR), surrogates of glycolysis and OXPHOS (Figure 21 and Figure 22), likely due to diminished mitochondrial function and elevated ROS levels (Figure 23)

In summary, these findings suggest that even within metastatic PDX HB cells, NEDP1 functions as a tumor suppressor.

### Restoration of NEDP1 Activity in Hepatoblastoma Murine Models attenuates Tumor Growth and Development

To evaluate the tumor-suppressive effects of NEDP1 in mouse models, an associated adenovirus encoding the recombinant mouse *Senp8* gene (HB-AAV-Senp8), the gene name of NEDP1 protein, or associated control adenovirus (HB-AAV-Gfp) was engineered for targeted liver overexpression. Hydrodynamic tail vein injections were performed on C57BL/6J 6-week-old mice to generate CTNNB1N90 YAP-1S127A HB mice model (Tao J, et al. Gastroenterology. 2014 Sep;147(3):690-701). The mice were monitored triweekly by echography for HB development. Nine weeks post-injection, once HB tumor has been formed the mice were divided into two groups based on image studies and subsequently received weekly treatments with either HB-AAV-*Gfp* or HB-*AAV-Senp8.*

Notably, NEDP1 activity in HB tumors was significantly lower compared to normal liver tissue. Overexpression of NEDP1 in tumors restored its activity to levels like those observed in healthy liver tissue (Figure 24), with these increases confirmed by qPCR analysis (Figure 25 A). Although there were no relevant changes in biochemical markers (Figure 25 B), histological analysis conducted by a pathologist showed that NEDP1 overexpression resulted in reduced tumor infiltration (Figure 25C). Additionally, immunohistochemistry revealed a significant reduction in NEDD8 levels following NEDP1 upregulation (Figure 25 D).

To determine the underlying cause of the observed results, the model was replicated in ^{Bio}NEDD8, whole-body genetically modified mice (Gonzalez-Rellan MJ, et al. Cell Metab. 2023 Sep;35(9):1630-1645.e5). The increase of NEDP1 in tumors was confirmed by qPCR and Western Blot (Figure 26 A and B). Similarly to C57BL/6J mouse model, ^{Bio}NEDD8 changes in tumor development were observed in these mice, as confirmed by histopathological analysis and H&E staining (Figure 26 C). No relevant alterations were observed in biochemical markers (Figure 26 D). Finally, immunohistochemistry revealed a significant reduction in NEDD8 levels following NEDP1 upregulation (Figure 26 E).

Subsequently, biotin pull-down assays were performed on ^{Bio}NEDD8 mice to investigate changes in the hepatic HB biotin-NEDDylated proteome in the absence or presence of NEDP1 overexpression. A proteomic analysis using LC-MS identified 974 proteins, with 34 exhibiting statistically significant differences between control HB-AAV-*Gfp* -treated tumors and those overexpressing NEDP1 (HB-AAV-*Senp8*). Figure 27 presents the volcano plot analysis of the hepatic NEDDylome in tumors with HB-AAV-*Senp8,* revealing significant protein modulation compared to the HB-AAV-*Gfp* control group. This analysis highlighted CAND1, which is associated with the NEDDylome proteome in HB and was significantly reduced in animals where NEDP1 was overexpressed. Given its role, alongside NEDD8, in maintaining protein homeostasis through the regulation of the SCF complex, CAND1 has been considered previously as a potential driver of HCC (Zhang H, et al. Clin Transl Med. 2023 Oct 14;13(10).).

Overall, NEDP1 overexpression in preclinical HB models reduces tumor size and progression while inducing NEDDylome reorganization, with CAND1 emerging as a key target in these preclinical mouse models.

### CAND1 as a Key Mediator of Hepatoblastoma Alterations Induced by NEDP1 Overexpression and DeNEDDylation

To confirm that CAND1 underwent NEDDylation in HB mice model, Western blot analyses were conducted on biotin pull-down extracts from ^{bio}NEDD8 mice (Figure 28 A). The results demonstrated that NEDDylated CAND1 was significantly elevated in HB-AAV-*Gfp* compared to healthy liver tissues. Conversely, in HB-AAV-*Senp8* mice, levels of NEDDylated CAND1 were markedly reduced. Additionally, Western blot quantification of liver extracts from both ^{bio}NEDD8 and C57BL/6J HB-AAV-*Gfp* mouse models (Figure 28 A and B) revealed an increase in total CAND1 levels relative to those in healthy livers, with a notable decrease observed following HB-AAV-*Senp8* overexpression.

To further validate these findings, immunoprecipitations with anti-NEDD8 antibodies were performed in the previously described HB cell lines (Figure 29). Western blot analysis revealed a significant upregulation of NEDDylated CAND1 in HB cell lines, whereas NEDP1 overexpression led to a marked reduction in its NEDDylated form. The absence of the NEDDylated form of CAND1 in the presence of NEDP1 is associated with a reduction in the total levels of this protein (Figure 29 and 30).

Based on these results, the inventors aimed to confirm that the antitumor effect observed with NEDP1 overexpression is linked to a reduction in CAND1. To achieve this, both CAND1 and NEDP1 were simultaneously overexpressed in HB cell lines (Figure 31). Elevated CAND1 levels abrogated the antitumor effect of NEDP1, leading to increased HB cell proliferation (Figure 32 A and B). Accordingly, the overexpression of CAND1 inhibited the apoptotic response mediated by NEDP1, as demonstrated by a reduction in caspase-3 levels (Figure 32 C and D).

These results indicate that both NEDDylated and total levels of CAND1 play a significant role in HB, mediating the antitumoral effects observed by NEDP1 overexpression, contributing to the phenotypic outcomes.

### CAND1 Levels are Associated with Hepatoblastoma Molecular and Clinical Variables of Poor-Prognosis and Patients Outcome

The analysis of HTA database of 32 HB patients and 20 non-tumoral (NT) samples revealed a significant upregulation of CAND1 in HB (Figure 33).

To further evaluate the implication of CAND1 expression in HB aggressiveness, the association of its expression with molecular variables such as C1/C2-Pure/C2B classification, epigenetic Epi-CA/CB subtypes, 14q32-gene signature and Molecular Risk Stratification (MRS) (Carrillo-Reixach J, et al. J Hepatol. 2020 Aug;73(2):328-41, Cairo S, et al. Cancer Cell. 2008 Dec;14(6):471-84; Hooks KB, et al. Hepatology. 2018 Jul 14;68(1):89-102) as well as with key clinical prognostic variables such as PRETEXT (PRE-Treatment EXTent of disease), multifocality, vascular invasion, presence of metastasis and CHIC (Children's Hepatic International Collaboration -Hepatoblastoma Stratification) was analyzed.

Patients were categorized into different groups based on their levels of CAND1 expression in tumor tissues, which were stratified according to the mean, median, 25th percentile, and 75th percentile of Log2 tumor expression. By segmenting patients in this way, the inventors aimed to identify potential thresholds of CAND1 expression that may be linked to tumor aggressiveness and clinical outcomes. Interestingly, overexpression of CAND1 (i.e. above percentile 75) was found to be associated with high-risk molecular features, such as the C2-pure subtype of the 16-gene signature (p=0.005) and the epigenetic Epi-CB subtype (p=0.040) (Table 2).

**Table 2 CAND1 Expression and Its Correlation with Tumor Aggressiveness in Human Patients. CAND1 gene expression correlation with clinical and molecular features of tumor aggressiveness obtained by HTA array. Chi-square and Fisher Exact tests were used as appropriate.**

| | | **Mean** | | **Median** | | **Percentile 25** | | **Percentile 75** | |
|---|---|---|---|---|---|---|---|---|---|
| | | **(value: 10.30)** | | **(value: 10.21)** | | **(value: 9,26)** | | **(value: 10.58)** | |
| | | **Lower** | **Higher** | **Lower** | **Higher** | **Lower** | **Higher** | **Lower** | **Higher** |
| **Age** | **< 8 years** | 16 | 12 | 14 | 14 | 7 | 21 | 22 | 6 |
| | **≥ 8 years** | 2 | 2 | 2 | 2 | 1 | 3 | 2 | 2 |
| | **p value** | 0.788 | | 0.999 | | 0.999 | | 0.217 | |
| **Gender** | **Male** | 7 | 7 | 6 | 8 | 3 | 11 | 8 | 6 |
| | **Female** | 11 | 7 | 10 | 8 | 5 | 13 | 16 | 2 |
| | **p value** | 0.530 | | 0.476 | | 0.681 | | **0.040** | |
| **PRETEXT** | **I / II / III** | 14 | 12 | 12 | 14 | 7 | 19 | 18 | 8 |
| | **IV** | 3 | 2 | 3 | 2 | 1 | 4 | 5 | 0 |
| | **p value** | 0.648 | | 0.508 | | 0.798 | | 0.292 | |
| **Metastasis** | **No** | 13 | 10 | 12 | 11 | 7 | 16 | 16 | 7 |
| | **Yes** | 5 | 4 | 4 | 5 | 1 | 8 | 8 | 1 |
| | **p value** | 0.960 | | 0.694 | | 0.256 | | 0.256 | |
| **Vascular invasion** | **No** | 11 | 8 | 10 | 9 | 5 | 14 | 14 | 5 |
| | **Yes** | 7 | 6 | 6 | 7 | 3 | 10 | 10 | 3 |
| | **p value** | 0.821 | | 0.719 | | 0.835 | | 0.835 | |
| **Multifocality** | **No** | 12 | 8 | 11 | 9 | 6 | 14 | 16 | 4 |
| | **Yes** | 6 | 6 | 5 | 7 | 2 | 10 | 8 | 4 |
| | **p value** | 0.581 | | 0.456 | | 0.399 | | 0.399 | |
| **CHIC-HS** | **VL/L/Int risk** | 13 | 7 | 12 | 8 | 7 1 | 13 | 15 | 5 |
| | **High risk** | 5 | 7 | 4 | 8 | | 11 | 9 | 3 |
| | **p value** | 0.198 | | 0.144 | | 0.092 | | 0.999 | |
| **16+VIM-gene signature** | **C1/C2B** | 15 | 6 | 13 | 8 | 7 | 14 | 19 | 2 |
| | **C2-Pure** | 3 | 8 | 3 | 8 | 1 | 10 | 5 | 6 |
| | **p value** | **0.017** | | 0.063 | | 0.133 | | **0.005** | |
| **Epigenetic subtype** | **Epi-CA** | 13 | 5 | 12 | 6 | 6 | 12 | 16 | 2 |
| | **Epi-CB** | 5 | 9 | 4 | 10 | 2 | 12 | 8 | 76 |
| | **p value** | **0.039** | | **0.033** | | 0.217 | | **0.040** | |
| **14q32 expression** | **Moderate** | 8 | 4 | 8 | 4 | 5 | 7 | 11 | 1 |
| | **Strong** | 10 | 10 | 8 | 12 | 3 | 17 | 13 | 7 |
| | **p value** | 0.358 | | 0.144 | | 0.092 | | 0.092 | |
| **MRS** | **Low Risk** | 8 | 3 | 8 | 3 | 5 | 6 | 10 | 1 |
| | **Intermediate risk** | 5 | 3 | 4 | 4 | 1 | 7 | 7 | 1 |
| | **High risk** | 5 | 8 | 4 | 9 | 2 | 11 | 7 | 6 |
| | **p value** | 0.222 | | 0.123 | | 0.152 | | **0.072** | |
| Abbreviations: CHIC-HS, Children's Hepatic International Collaboration Hepatoblastoma Stratification; VL: Very-low; L: Low; Int: Intermediate; PRETEXT, PREtreatment EXTent of the disease; MRS: Molecular Risk Stratification. Data published by Carrillo-Reixach et al, J Hepatology (2020). | | | | | | | | | |

Next, it was assessed whether the gene expression of CAND1 was related to patient's survival. Table 3 summarizes the results of the event-free survival (EFS) and overall survival (OS) univariate analysis for the different cut-offs established. Notably, very high gene expression levels of CAND1 (cut-off: 75th percentile) were significantly associated with poorer prognosis (log rank=0.033). Patients with high CAND1 expression levels showed only a 50% OS rate compared to those with low levels, who exhibited an 83% OS rate (Figure 34).

**Table 3. CAND1 association with HB patient's survival. Event-free survival (EFS) and overall survival (OS) Log-rank test of HB patients categorized according to CAND1 gene expression levels.**

| | **EFS** | | | | **OS** | | | |
|---|---|---|---|---|---|---|---|---|
| **Cut-off** | **pScore LogRank** | **HR** | **CI5** | **CI95** | **pScore LogRank** | **HR** | **CI5** | **CI95** |
| **Mean** | 0.0899 | 2.745 | 0.8168 | 9.222 | 0.2583 | 2.231 | 0.5497 | 9.052 |
| **Median** | 0.0905 | 2.958 | 0.9058 | 9.661 | 0.4972 | 1.633 | 0.4084 | 6.530 |
| **Percentile 25th** | 0.1466 | 4.033 | 1.093 | 14-89 | 0.3737 | 2.502 | 0.5189 | 12.07 |
| **Percentile 75th** | 0.0728 | 2.874 | 0.5868 | 14.08 | **0.0334** | **3.969** | **0.7048** | **22.35** |
| Abbreviations: HR, hazard ratio; CI, confidence interval | | | | | | | | |

These results indicate that CAND1 is significantly overexpressed in HB tumors compared to adjacent NT tissues. High CAND1 expression is associated with poor-prognosis molecular and clinical variables, including aggressive subtypes and adverse clinical features like vascular invasion. Elevated CAND1 levels were linked to worse patient outcomes, particularly lower overall survival rates.

### Potential Role of CAND1 in Hepatocellular Carcinoma Progression

Having previously elucidated the role of CAND1 in HB prognosis, these findings were extended to investigate their potential involvement in hepatocellular carcinoma (HCC). Recent data suggests that CAND1 mRNA expression may significantly affect the prognosis of patients with HCC. Specifically, variations in the mRNA expression of CAND1, as demonstrated by the Kaplan-Meier survival curve (Figure 35), suggest a potential link between its expression levels and patient survival in HCC. Higher CAND1 expression is associated with significantly reduced survival, indicating that dysregulation of CAND1 could play a critical role in HCC progression, as it does in HB.

This finding suggests that CAND1 dysregulation may play a crucial role in the progression of HCC, warranting further investigation into its potential as a therapeutic target or prognostic marker in this cancer.

## Claims

1. An agent capable of inhibiting the expression of Cullin Associated and Neddylation Dissociated 1 (*CAND1*) and/or inhibiting the activity of the protein encoded by said gene for use in the treatment and/or prevention of hepatoblastoma or hepatocellular carcinoma.

2. The agent for use according to claim 1, wherein the agent is selected from the group consisting of:
- a neutralising antibody or an antigen-binding fragment thereof,
- a small interfering RNA (siRNA),
- a small molecule,
- a phosphorodiamidate morpholino oligomer (PMO),
- a short hairpin RNA (shRNA),
- an antisense oligonucleotide and
- a polynucleotide sequence coding for NEDD8-specific Protease 1(NEDP1).

3. The agent for use according to claim 2, wherein the siRNA has a sense strand defined by SEQ ID NO: 2 and an antisense strand defined by SEQ ID NO: 3.

4. The agent for use according to claims 2 or 3, wherein the siRNA is conjugated to a triantennary N-acetylgalactosamine (GalNAC).

5. The agent for use according to any one of claims 1 to 4, wherein the hepatoblastoma or the hepatocellular carcinoma is **characterized by** having high levels of NEDDylation.

6. An *in vitro* method for the diagnosis of a hepatoblastoma or a hepatocellular carcinoma in a subject, the method comprising:
(i) determining *CAND1* and/or *NEDP1* expression levels in a liver biopsy sample from a subject and
(ii) comparing these levels with a reference value, wherein increased expression levels of *CAND1* and/or decreased expression levels of NEDP1 compared with the reference value are indicative that the subject suffers from hepatoblastoma.

7. An *in vitro* method for determining the prognosis of a patient with a hepatoblastoma or a hepatocellular carcinoma, the method comprising:
(i) determining *CAND1* and/or *NEDP1* expression levels in a liver biopsy sample from a subject and
(ii) comparing these levels with a reference value, wherein:
- if the expression levels of CAND1 are increased compared to a reference value and/or,
- if the expression levels of NEDP1 are decreased compared to a reference value
the patient has a poor prognosis.

8. The method according to claim 7, wherein poor prognosis refers to low overall survival and low event-free survival.

9. The method according to any one of claims 6 to 8, wherein the expression levels of *CAND1* are determined by measuring mRNA transcript levels.

10. The method according to claim 9, wherein mRNA transcript levels are determined by means of human transcriptome array analysis.

11. The method according to any one of claims 6 to 8, wherein the expression levels of *CAND1* and/or *NEDP1* are determined by measuring protein levels.

12. The method according to claim 11, wherein the protein levels of CAND1 and/or NEDP1 are determined by means of immunoblotting or mass spectrometry.

13. An *in vitro* method for determining the predisposition of a subject to developing hepatoblastoma or hepatocellular carcinoma, the method comprising:
(i) determining the deNEDDylation activity in a liver biopsy sample and
(ii) comparing these levels with a reference value, wherein the decreased levels of deNEDDylation activity of the sample compared to a reference value are indicative of increased chances of developing a hepatoblastoma or hepatocellular carcinoma.

14. The method according to claim 13, wherein step (i) further comprises:
a) generating a lysate from a liver biopsy sample from the subject,
b) contacting the liver biopsy lysate, which contains NEDP1, with a NEDP1 substrate comprising a Neural precursor cell Expressed, Developmentally Down-regulated 8 (NEDD8) molecule conjugated to a quenched fluorescent molecule, thereby allowing the hydrolysis of the linkage between NEDD8 and the quenched fluorescent molecule due to the deneddylation activity of NEDP1 present in the liver biopsy lysate and,
c) detecting the fluorescence signal from the unquenched fluorescent molecule as a result of the deNEDDylation.
